# EUROPEAN PATENT APPLICATION

(11) **EP 3 543 694 A1**
(43) Date of publication of application: **25.09.2019**
(21) Application number: 19150937.1
(22) Date of filing: 14.03.2014
(51) Int. Cl.: G01N 33/53

(54) **METHODS AND COMPOSITIONS FOR DIAGNOSING PREECLAMPSIA**

(30) Priority: 14.03.2013 US 201361785934 P
(62) Divisional of application: 14768554.9
(71) Applicant: IMMUCOR GTI DIAGNOSTICS, INC., Norcross GA 30357 (US)
(72) Inventor: Roedder, Sike, Mountain View, CA 94043 (US); Abdullah, Isha, Mountain View, CA 94043 (US); Sarwal, Minnie M., Mountain View, CA 94043 (US)
(74) Representative: Hoffmann Eitle

(57) **Abstract**

Provided herein are methods, compositions, and kits for using binding agents to detect the presence of PEE proteins and/or PEE autoantibodies in a biological sample from a pregnant woman. Such methods and compositions are useful for predicting the onset of preeclampsia, monitoring the progression of preeclampsia, monitoring the regression of preeclampsia, assessing efficacy of treatment for preeclampsia, identifying a sub-population of patients who should be treated for preeclampsia and/or identifying a sub-population of patients who should be monitored for preeclampsia symptoms.

## Description

### CROSS-REFERENCE TO RELATED APPLICATION

This application claims the priority benefit to U.S. Provisional Patent Application Serial No. 61/785,934, filed March 14, 2013, the entire content of which is incorporated herein by reference.

### BACKGROUND OF THE INVENTION

Preeclampsia (PEE) is a disorder that occurs during pregnancy and can lead to morbidity and mortality in both the mother and the fetus. This condition is prevalent in pregnant mothers both in the United States (U.S.) and abroad. PEE impacts 5-15% of all births worldwide. Worldwide, PEE is responsible for 20% of the 13 million preterm births each year. In the U.S. alone, 100,000 of the total annual 500,000 premature births are a result of PEE annually. Worldwide, of the 30 million intra-uterine growth retarded infants born each year, 15% (4.5 million) are associated with PEE. Approximately 0.5 million babies worldwide, and 10,500 babies in the U.S. die from maternal PEE each year. Stillbirths from PEE in the U.S. are approximately 2200 each year. Further compounding these numbers are that worldwide, PEE is poorly reported.

PEE involves pregnancy-induced hypertension in which protein is often observed in a subject's urine. At present, PEE is diagnosed by an elevation of the expectant mother's blood pressure, usually only after the 20^{th} week of pregnancy, combined with the appearance of excessive protein in her urine. PEE is currently defined by an elevation in blood pressure (>140/90 mm Hg) and protein in the urine (>300 mg/24 hours) occurring in the second half of pregnancy in women without a history of high blood pressure, kidney disease, diabetes, or other significant disease. PEE may also include a myriad of other abnormalities.

There is no precise way to diagnose if the condition exists, and so the possibility of PEE is always considered for women displaying those particular symptoms beyond 20 weeks gestation. PEE has proven particularly difficult to diagnose because its symptoms mimic many other diseases and includes symptoms such as headaches, abdominal pain, visual disturbances, confusion, anxiety, shortness of breath, nausea, and vomiting. If left undiagnosed, or diagnosed too late, there can be a serious impact on both women and their babies. Moreover, PEE can progress from mild to severe rapidly. Serious signs of the disease include high blood pressure, risk of brain injury, impaired kidney and liver function, blood clotting problems, pulmonary edema, and even seizures. Further adding to the complexity of diagnosis, some women exhibit preexisting hypertension, which is often difficult to discern from the onset of PEE. To date, there is no effective way to diagnose this potentially fatal condition prior to the onset of symptoms.

PEE can result in maternal complications such as eclampsia and the HELLP syndrome (hemolysis, elevated liver enzymes, and lowered platelets). PEE can result in fetal complications such as prematurity, intrauterine growth restriction; acidosis, ongoing life challenges such as learning disorders, cerebral palsy, epilepsy, blindness, and deafness, or can even result in fetal death.

Early detection, diagnosis and/or prediction of the onset of PEE would prevent or delay many adverse maternal and fetal outcomes of PEE. The early molecular abnormalities present in preeclamptic women are only now being recognized but accurate and/or sensitive ways of diagnosing the women in need for treatment is still needed. Therefore, there is an unmet need for accurate testing for the early detection of mothers that will likely experience PEE. The invention described herein addresses this need and provides additional benefits as well.

Throughout the specification, various references, publications, patents, and/or patent applications are cited. These references, publications, patents, and/or patent applications are incorporated by reference in their entirety for all purpose uses.

### BRIEF SUMMARY OF THE INVENTION

The invention described herein provides, *inter alia,* methods, compositions, and kits useful for identifying the risk of developing preeclampsia (PEE), predicting the onset of PEE, monitoring the progression of PEE, monitoring the regression of PEE, identifying a sub-population of patients who should be treated for PEE or continue to be treated for PEE, assessing efficacy of treatment for PEE in pregnant women, and/or identifying a sub-population of patients who should be monitored for PEE symptoms.

In one aspect, the invention provides a method for detecting the risk of developing preeclampsia in a pregnant woman, the method comprising contacting a biological sample from the woman with a binding agent and detecting the binding of the binding agent to at least three PEE proteins present in the sample, wherein the binding agent binds the PEE proteins with a K_{d} of 10-¹² M to 10⁻⁵ M, and wherein the binding of the binding agent to the PEE proteins in the sample is increased as compared to the binding of the binding agent to the PEE proteins in a biological sample from a healthy pregnant woman in the same trimester, whereby the increase in binding indicates the risk of developing preeclampsia to at least a 80% degree of accuracy. In some embodiments, the increase in binding indicates the risk of developing preeclampsia to at least a 90% degree of accuracy. In other embodiments, the increase in binding indicates the risk of developing preeclampsia to at least a 95% degree of accuracy. In one embodiment, the method is used for predicting the onset of preeclampsia, monitoring the progression of preeclampsia, monitoring the regression of preeclampsia, assessing efficacy of treatment for preeclampsia, identifying a sub-population of patients who should be treated for preeclampsia or identifying a sub-population of patients who should be monitored for preeclampsia symptoms. In one embodiment the at least three PEE proteins are selected from the PEE proteins listed in Table 1. In another embodiment, the at least three PEE proteins are selected from the PEE proteins listed in Table 2. In another embodiment, the at least three PEE proteins are selected from the combinations of PEE proteins in Table 3. In another embodiment, the binding agent further binds yet another PEE protein selected from Table 1 or Table 2. In certain embodiments, the binding agent binds no more than 48 PEE proteins. In certain embodiments, the woman is in the third trimester of her pregnancy. In certain embodiments, the woman is in the second trimester of her pregnancy. In certain embodiments, the woman is in the first trimester of her pregnancy. In some embodiments, the biological sample is a non-fetal maternal sample. In some embodiments, the biological sample is urine, blood, or placental tissue. In some embodiments, the binding agent comprises a mixture of individual antibodies to the PEE proteins GSN, THBS1 and PRG2. In yet other embodiments, the binding agent further binds an autoantibody present in the sample. In some such embodiments, the binding agent further binds an autoantibody selected from Table 4or Table 5. In one specific embodiment, the binding agent binds PEE autoantibodies to CSNK1D, CUEDC1 and ZRANB2. In another specific embodiment, the binding agent binds PEE autoantibodies to CSNK1D, SULT4A1 and Junction Plakoglobin. In one embodiment of the method provided herein, the binding is carried out by an immunoassay. In another embodiment, the immunoassay is an enzyme-linked immunosorbent assay. In another embodiment, the immunoassay comprises beads. In another embodiment, the immunoassay comprises magnetic particles. In another embodiment, the immunoassay does not comprise magnetic particles. In another embodiment, the binding agent is a nucleoprotein comprising protein binding sites.

In another aspect, the invention provides a method for detecting the risk of developing preeclampsia in a pregnant woman, the method comprising contacting a biological sample from the woman with a binding agent, and detecting the binding of the binding agent to an PEE autoantibody present in the sample, wherein the binding agent binds the PEE autoantibody with a K_{d} of 10-¹² M to 10⁻⁵ M, and wherein the binding of the binding agent to the PEE autoantibody in the sample is changed as compared to the binding of the binding agent to the PEE autoantibody in a biological sample from a healthy pregnant woman in the same trimester, whereby the change in binding indicates the risk of developing preeclampsia to at least a 80% degree of accuracy. In one embodiment, the increase in binding indicates the risk of developing preeclampsia to at least a 90% degree of accuracy. In another embodiment, the increase in binding indicates the risk of developing preeclampsia to at least a 95% degree of accuracy. In one embodiment, the PEE autoantibody is selected from Table 4 or Table 5. In another embodiment, the binding agent comprises a protein. In another embodiment, the binding agent comprises a protein array. In another embodiment, the binding of the binding agent to the PEE autoantibody in the sample is increased as compared to the binding of the binding agent to the autoantibody in a biological sample from a healthy pregnant woman. In another embodiment, the binding of the binding agent to the PEE autoantibody in the sample is decreased as compared to the binding of the binding agent to the autoantibody in a biological sample from a healthy pregnant woman. In another embodiment, the binding agent binds PEE autoantibodies to CSNK1D, CUEDC1 and ZRANB2. In another embodiment, the binding agent binds PEE autoantibodies to CSNK1D, SULT4A1 and Junction Plakoglobin. In another embodiment, the binding agent further binds a PEE protein selected from Table 1or Table 2. In another embodiment, the binding agent further binds a PEE protein selected from GSN, THBS1 or PRG2. In another embodiment, the binding agent binds no more than 48 PEE proteins. In one embodiment the binding agent comprises a protein. In another embodiment, the binding agent comprises a protein comprising a label. In a specific embodiment, the label can be a fluorescent label. In one embodiment, the woman is in the third trimester of her pregnancy. In one embodiment, the woman is in the second trimester of her pregnancy. In one embodiment, the woman is in the first trimester of her pregnancy. In another embodiment, the biological sample is a non-fetal maternal sample. In another embodiment, the biological sample is urine, blood, or placental tissue. In another embodiment, the binding is carried out by an immunoassay. In another embodiment, the immunoassay is an enzyme-linked immunosorbent assay. In another embodiment, the immunoassay comprises beads. In another embodiment, the immunoassay comprises magnetic particles. In another embodiment, the immunoassay does not comprise magnetic particles. In one embodiment the binding agent comprises an antibody. In another embodiment, the binding agent comprises a nucleoprotein. In yet another embodiment, the binding agent comprises a peptide. In another embodiment, the binding agent comprises a fluorescent label. In another embodiment, the binding agent comprises a labeled antibody.

In yet another aspect, the invention provides a method for detecting the risk of developing preeclampsia in a pregnant woman, the method comprising contacting a biological sample from the woman with a binding agent, detecting the binding of the binding agent to at least one PEE protein and at least one PEE autoantibody present in the sample, wherein the binding agent binds the at least one PEE protein with a K_{d} of 10-¹² M to 10⁻⁵ M, wherein the binding agent binds the at least one PEE autoantibody with a K_{d} of 10-¹² M to 10⁻⁵ M, and wherein the binding of the binding agent to the at least one protein and the at least one PEE autoantibody in the sample is changed as compared to the binding of the binding agent to the at least one PEE protein and at least one PEE autoantibody in a biological sample from a healthy pregnant woman in the same trimester, whereby the change in binding indicates the risk of developing preeclampsia to at least a 80% degree of accuracy. In one embodiment, the increase in binding indicates the risk of developing preeclampsia to at least a 90% degree of accuracy. In another embodiment, the increase in binding indicates the risk of developing preeclampsia to at least a 95% degree of accuracy. In one embodiment, the method is further used for predicting the onset of preeclampsia, monitoring the progression of preeclampsia, monitoring the regression of preeclampsia, or assessing efficacy of treatment for preeclampsia. In one embodiment, the PEE autoantibody is selected from Table 4 or Table 5. In one embodiment, the binding of the binding agent to the PEE autoantibody in the sample is increased as compared to the binding of the binding agent to the autoantibody in a biological sample from a healthy pregnant woman. In another embodiment, the binding of the binding agent to the PEE autoantibody in the sample is decreased as compared to the binding of the binding agent to the autoantibody in a biological sample from a healthy pregnant woman. In one embodiment, the PEE protein is selected from Table 1 or Table 2. In another embodiment, the binding agent further binds a protein selected from GSN, THBS1 or PRG2. In one embodiment, the binding agent binds more than one PEE protein. In one specific embodiment, the binding agent binds the PEE proteins GSN, THBS1 and PRG2. In one embodiment, the binding agent binds no more than 48 PEE proteins. In one embodiment, the binding agent binds more than one PEE autoantibody. In one specific embodiment, the binding agent binds PEE autoantibodies to CSNK1D, CUEDC1 and ZRANB2. In another specific embodiment, the binding agent binds PEE autoantibodies to CSNK1D, SULT4A1 and Junction Plakoglobin. In one embodiment, the binding agent comprises a labeled antibody and a labeled protein. In a particular embodiment the label is a fluorescent label. In one embodiment, the woman is in the third trimester of her pregnancy. In another embodiment, the woman is in the second trimester of her pregnancy. In yet another embodiment, the woman is in the first trimester of her pregnancy. In one embodiment, the biological sample is a non-fetal maternal sample. In one embodiment, the biological sample is urine, blood, or placental tissue. In one embodiment, the binding is carried out by an immunoassay. In one specific embodiment, the immunoassay is an enzyme-linked immunosorbent assay. In another specific embodiment, the immunoassay comprises beads. In another specific embodiment, the immunoassay comprises magnetic particles. In one specific embodiment, the immunoassay does not comprise magnetic particles.

In another aspect, the invention provides a composition comprising one or more solid surfaces comprising binding agents for GSN, THBS1 and PRG2.

In another aspect, the invention provides a composition comprising one or more solid surfaces comprising binding agents for CSNK1D, CUEDC1 or ZRANB2 autoantibodies.

In another aspect, the invention provides a composition comprising one or more solid surfaces comprising binding agents for CSNK1D, SULT4A1 or Junction Plakoglobin autoantibodies.

In another aspect, the invention provides a diagnostic assay kit comprising: (a) reagents for detecting GSN, THBS1 and PRG2 in a biological sample from a pregnant woman; (b) a composition comprising one or more solid surfaces that contain one or more binding agents for GSN, THBS1 and PRG2; and (c) instructions for use of the assay.

In another aspect, the invention provides a diagnostic assay kit comprising: (a) reagents for detecting an autoantibody in a biological sample from a pregnant woman; (b) a composition comprising one or more solid surfaces that contain one or more binding agents for the PEE autoantibody; and (c) instructions for use of the assay. In one embodiment, the composition of the kit comprises a solid surface capable of binding the CSNK1D, CUEDC1 and ZRANB2 PEE autoantibodies. In another embodiment, the composition of the kit comprises a solid surface capable of binding the CSNK1D, SULT4A1 and Junction Plakoglobin PEE autoantibodies.

### BRIEF DESCRIPTION OF THE DRAWINGS

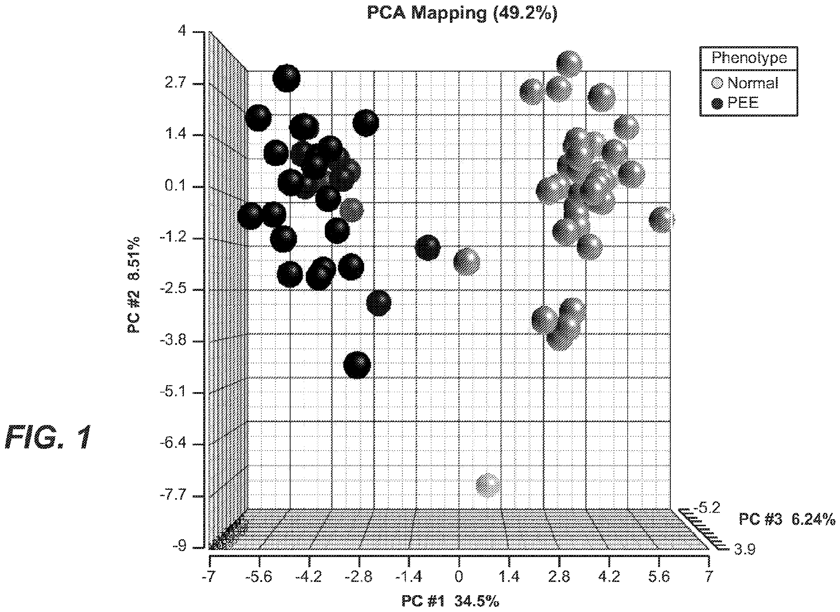

Figure 1 is a graphical representation of a principal component analysis of 48 PEE proteins that differentiate serum proteins in PEE (p<0.001 following ANOVA) when compared to sera from normal healthy pregnant women. The axes represent the eigenvectors of the covariance matrix scaled by the square root of the corresponding eigenvalue for the spectral counts of the 48 PEE proteins across each of the patients in the study (n=35 normal pregnancy, n=25 pregnancy with PEE). Also see Table 1.

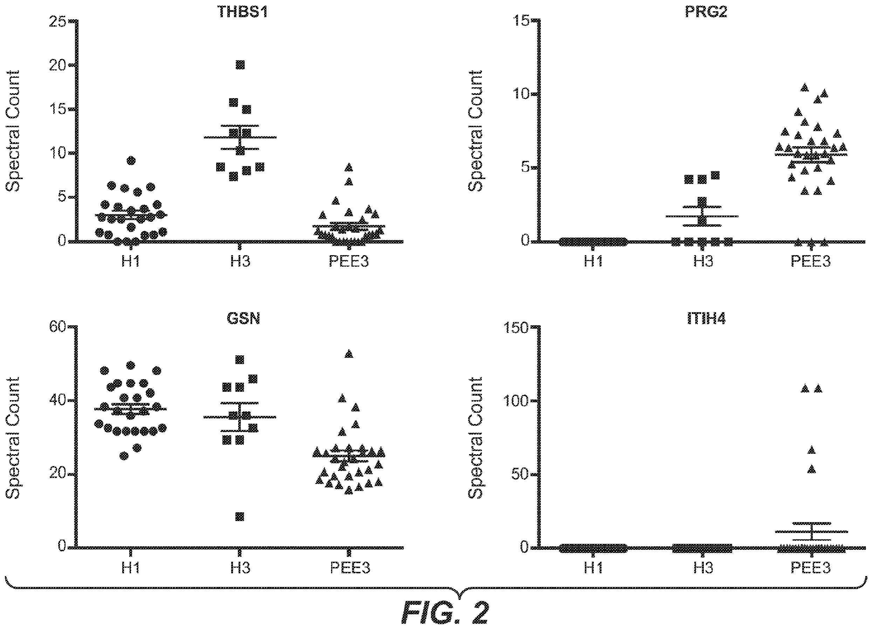

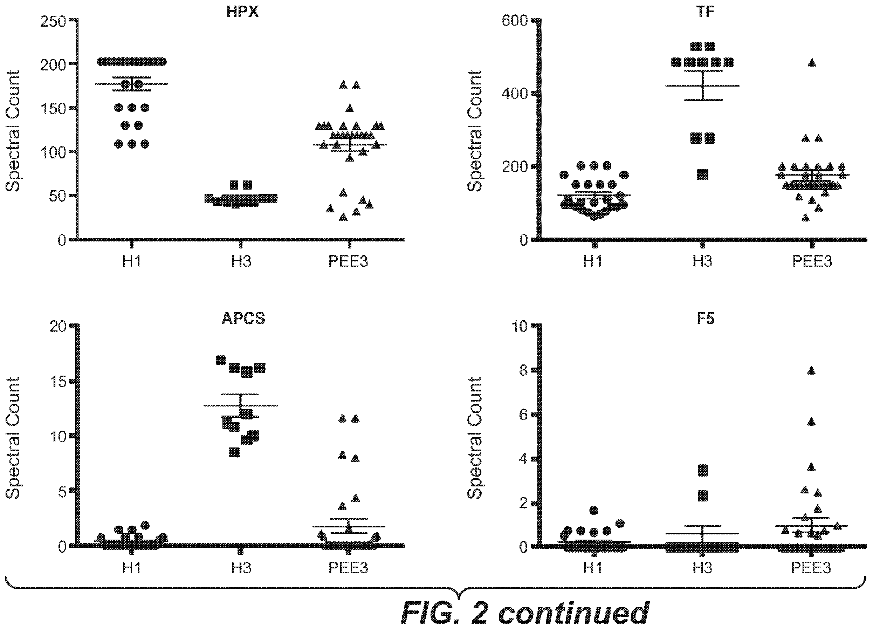

Figure 2 shows scatter plots for 8 proteins highly predictive of PEE. These 8 PEE proteins are a subset of the 48 PEE proteins in Figure 1: THBS1, PRG2, GSN, ITIH4, HPX, TF, APCS, and F5. Displayed are the mean spectral count values plus the SEM for each protein from healthy and PEE samples. Randomized combinations of three of these 8 proteins yield an average AUC of 0.95 or greater. Also see Table 2.

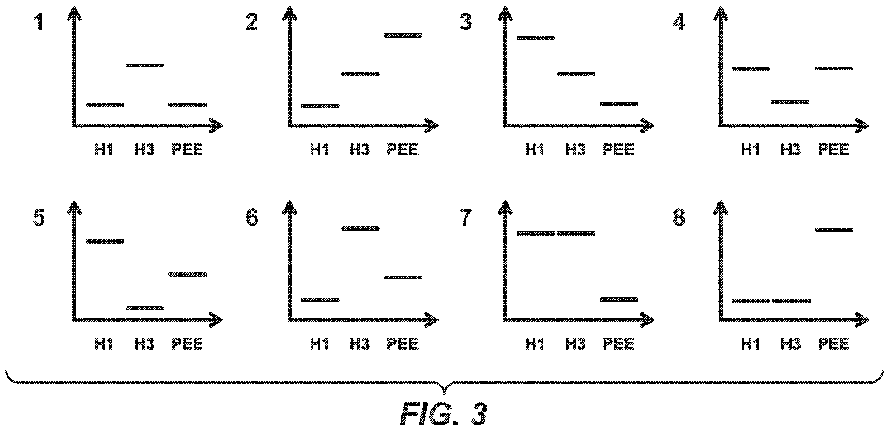

Figure 3 is a protein expression scheme for a biomarker pattern analysis to identify groups of proteins that show comparable patterns across trimesters. Identified patterns of proteins correspond to the column labeled "Biomarker Pattern Analysis" in Tables 1 and 2.

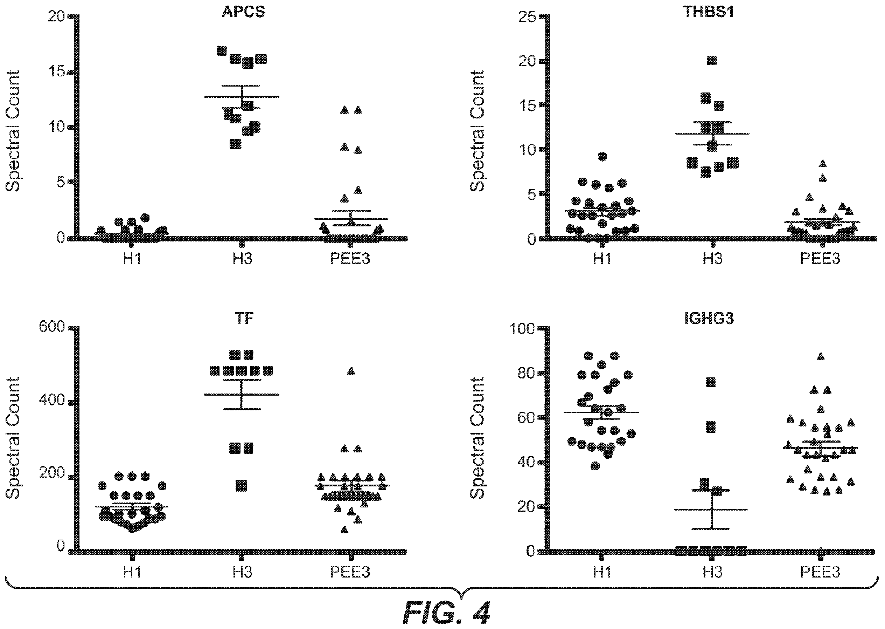

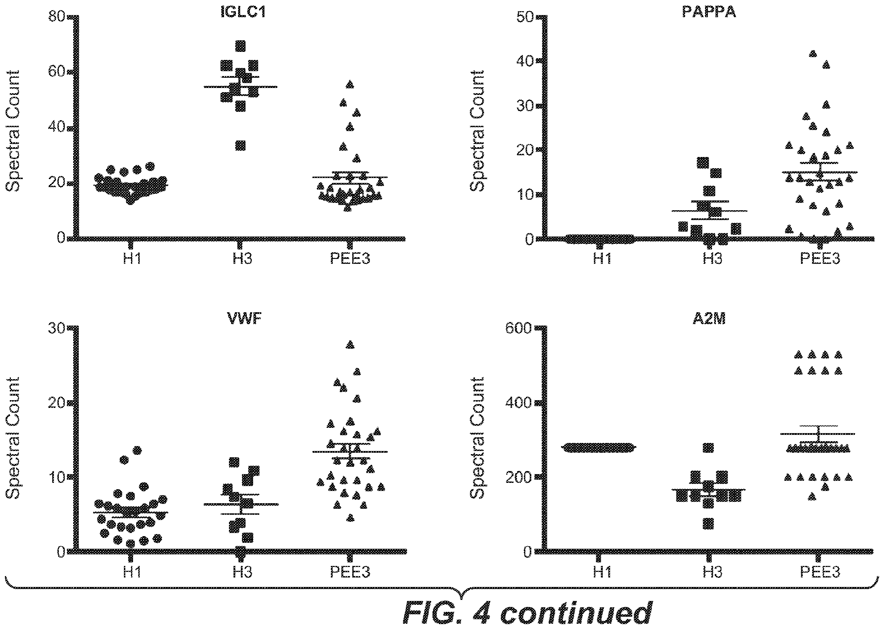

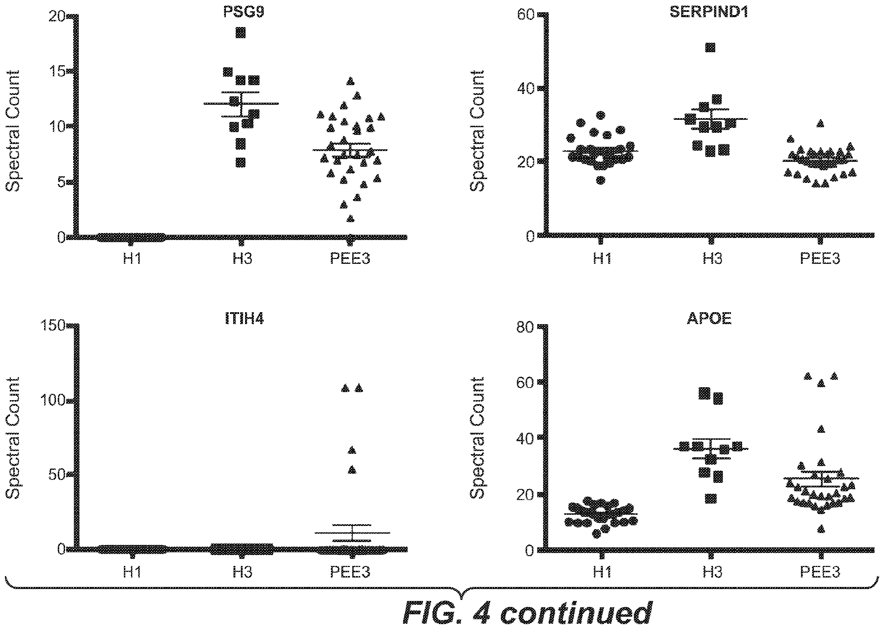

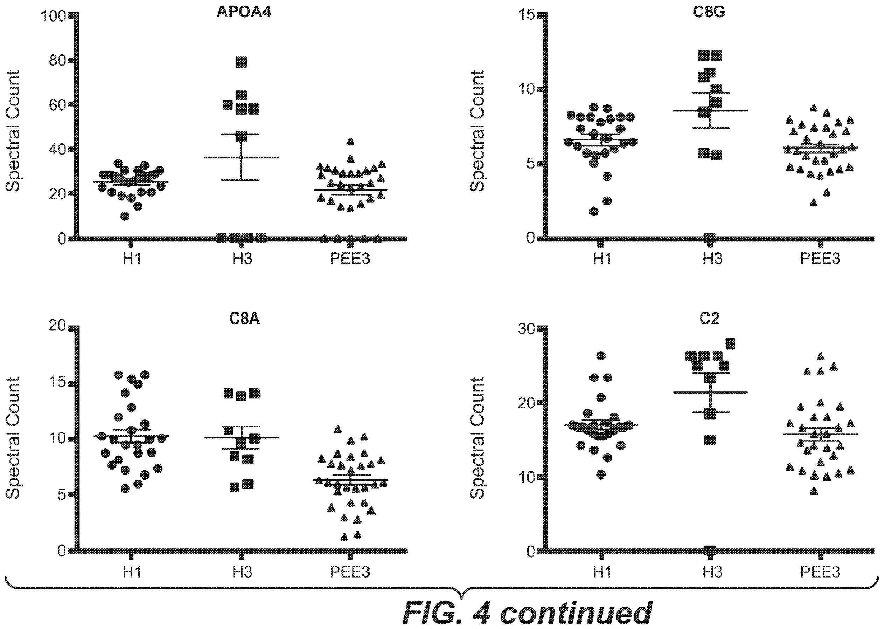

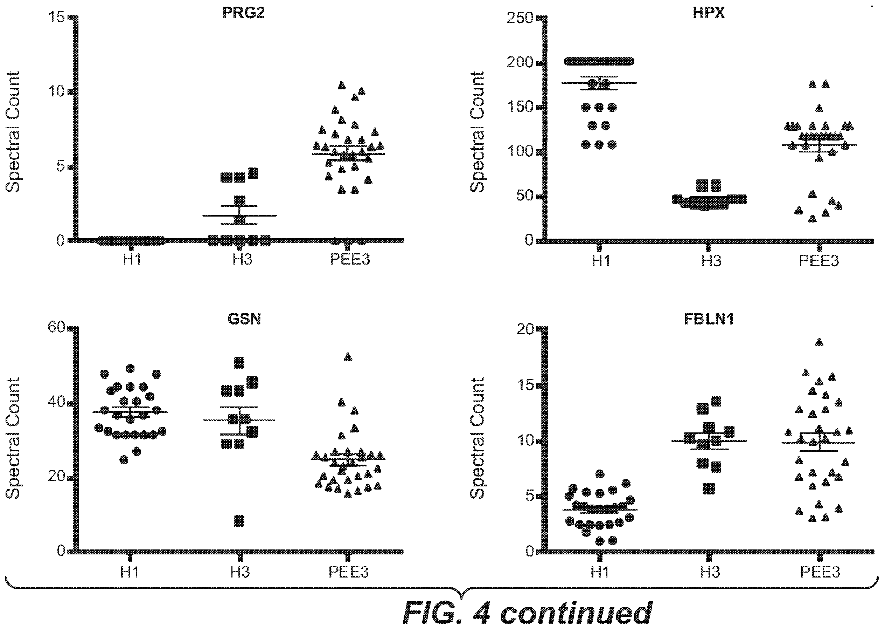

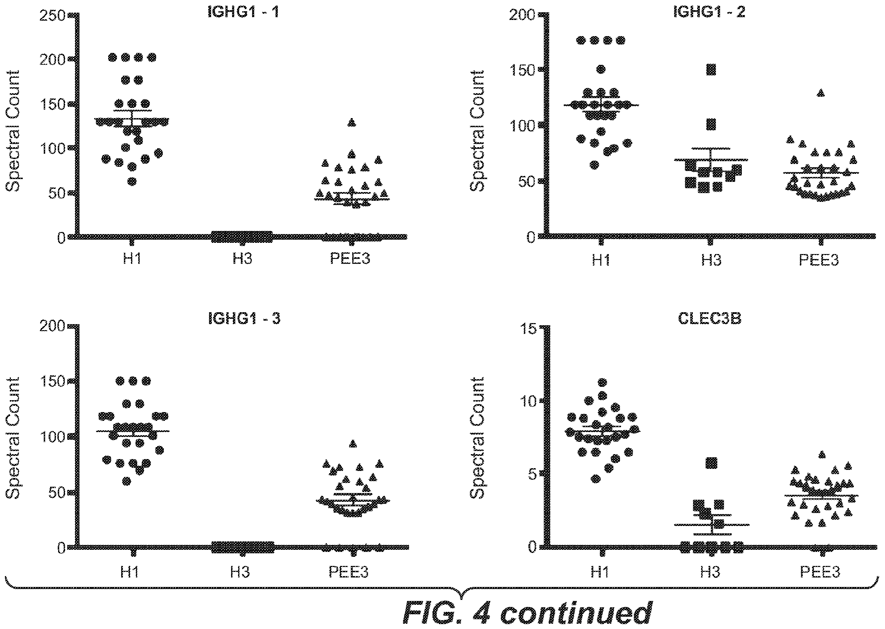

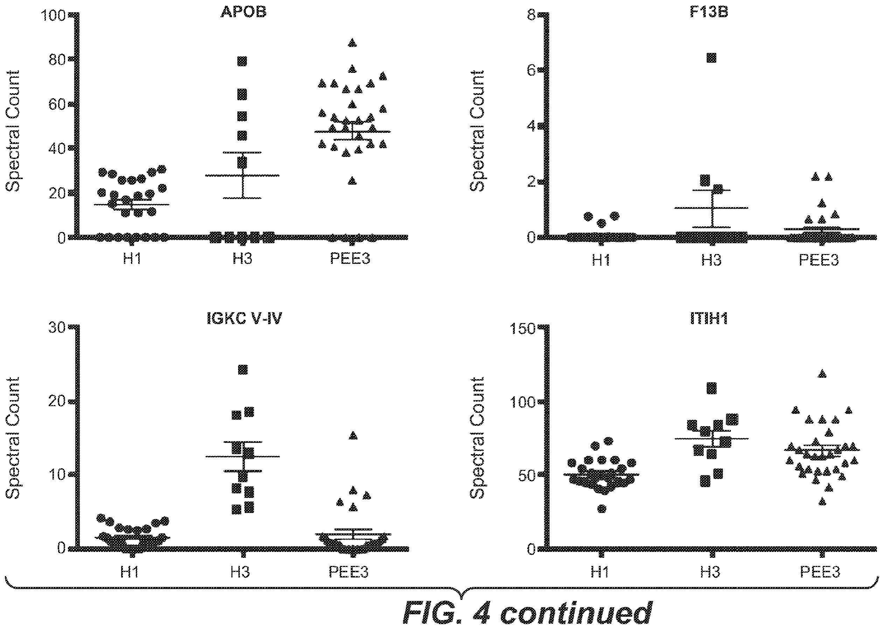

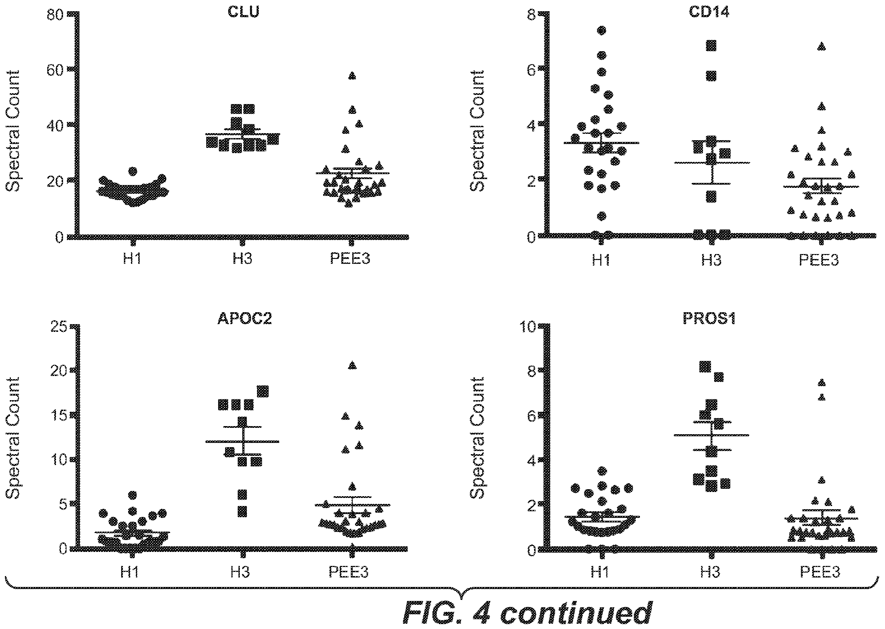

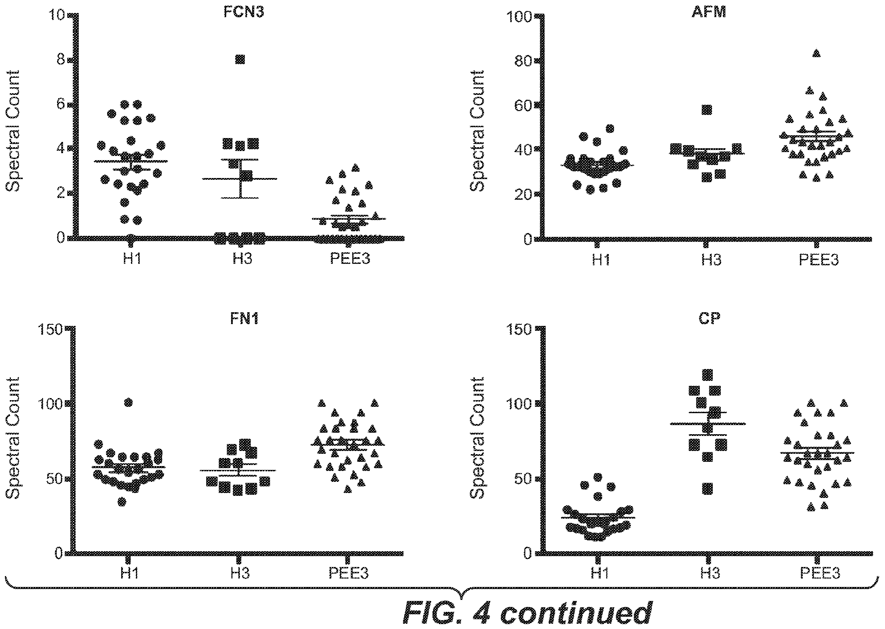

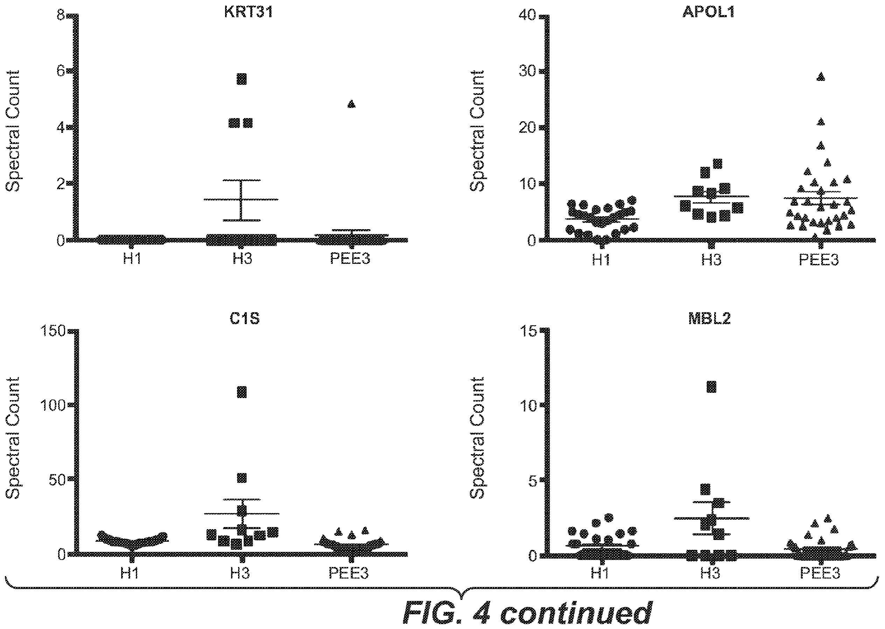

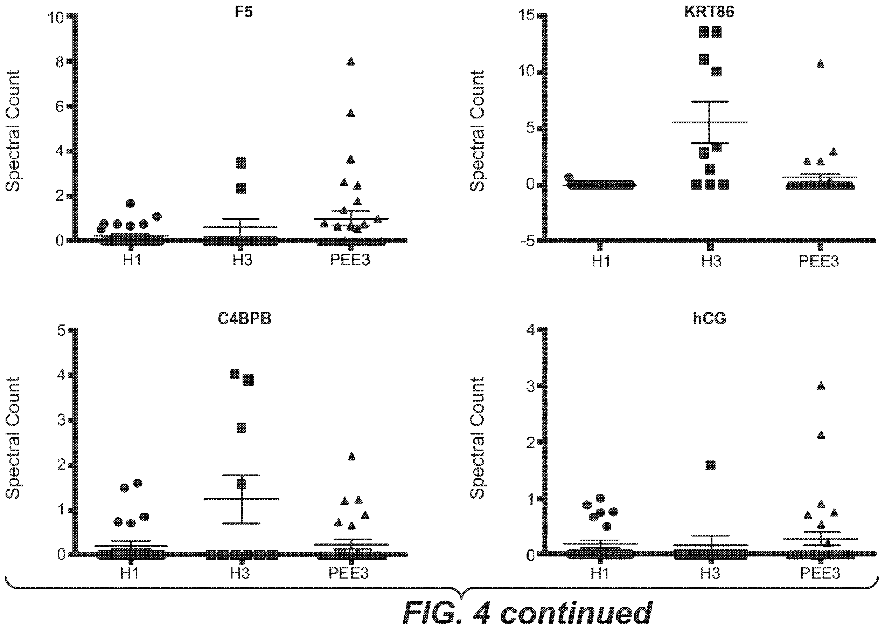

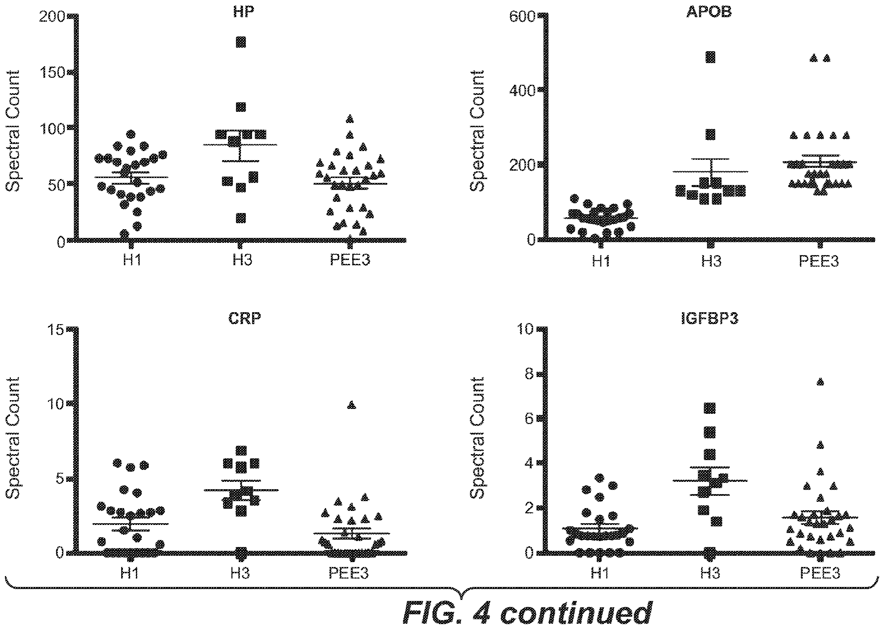

Figure 4 shows scatter plots of the individual spectral counts for each patient for the set of 48 PEE proteins in Table 1; displayed are mean spectral counts plus SEM for each protein.

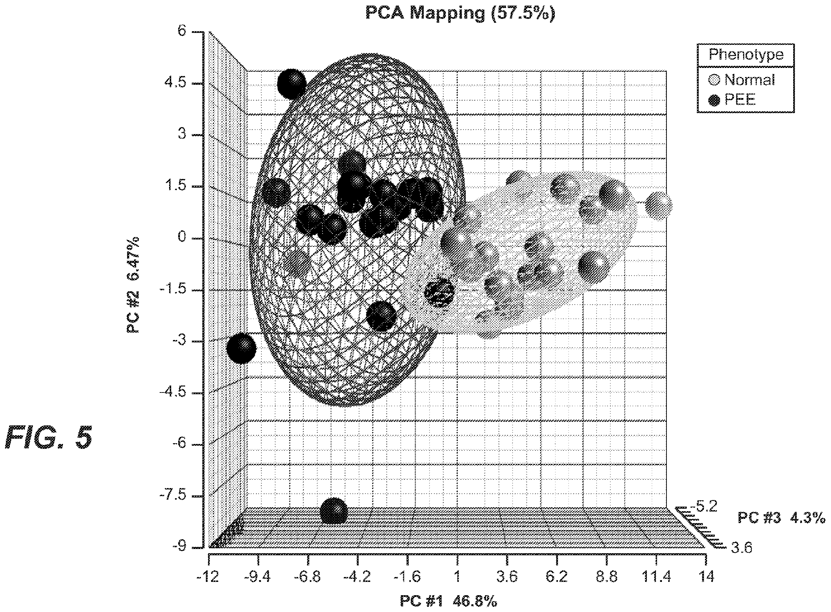

Figure 5 is a graphical representation of a principal component analysis following ANOVA of 75 PEE autoantibodies, with a false discovery rate adjusted p-value of qFDR<0.05 and a minimum fold change difference between Normal and PEE of 1.5, found to be differentially measured in serum samples from healthy pregnant women and pregnant women with PEE. Also see Table 4.

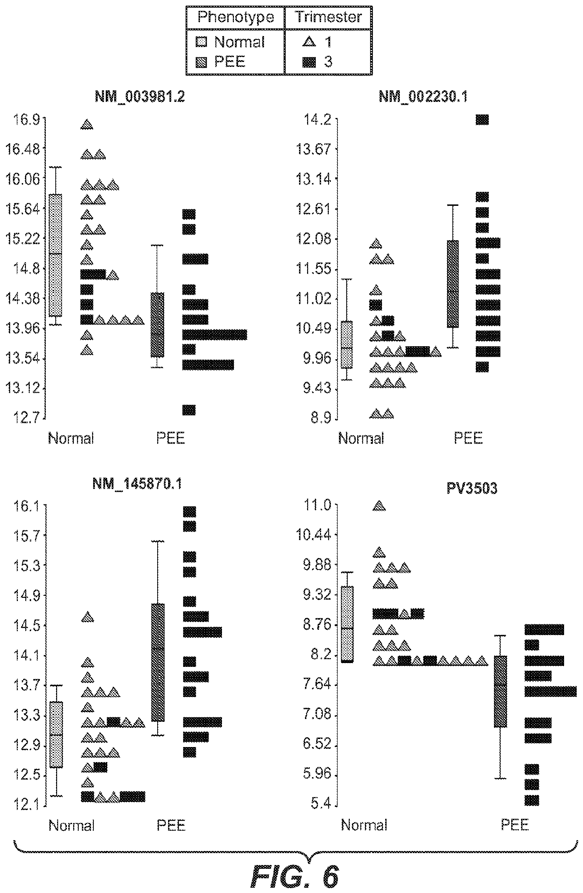

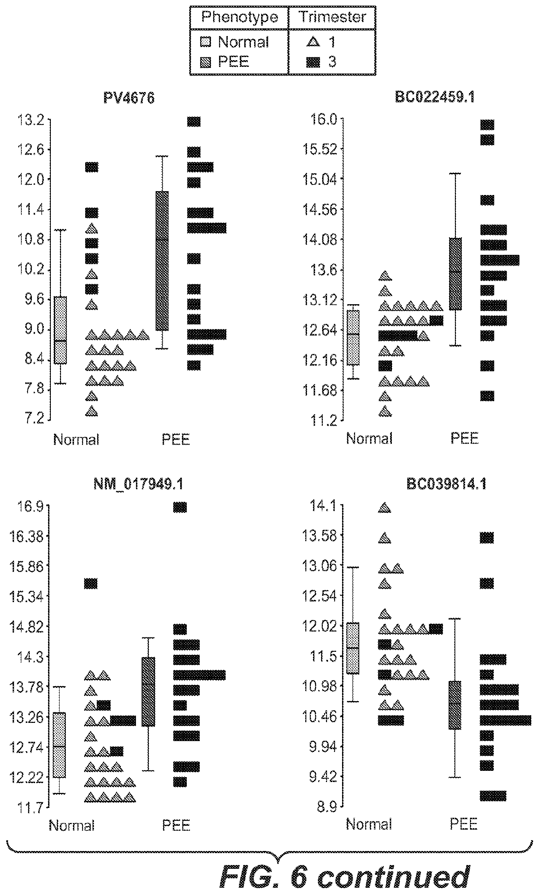

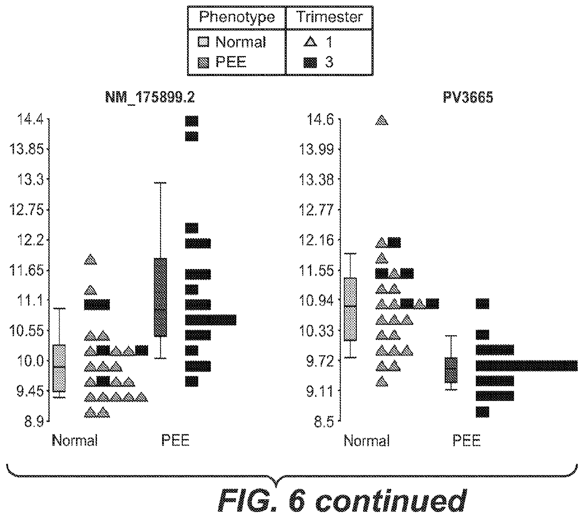

Figure 6 shows scatter plots of the individual mean fluorescent intensity (MFI) counts for each patient for a subset of 10 PEE autoantibodies from the set of 75 PEE autoantibodies (Figure 5 and Table 5) which were significantly different (qFDR<0.05; fold change >1.5) in sera taken from pregnant women with and without preeclampsia. Displayed are the mean MFI counts plus SEM of each autoantibody in serum samples from 25 healthy pregnant women (Normal) and 20 pregnant women with PEE. The light gray vertical bar on the left of each graph shows normal pregnant women in the first trimester (triangles) or the third trimester (rectangles). The dark gray vertical bar on the right of each graph shows the PEE women in the third trimester (rectangles). Biomarkers are chosen that can measured in normal pregnancy in the first trimester, with minimal overlap in the third trimester and with a significant (qFDR <0.05) increase or decrease in PEE over normal pregnancy with fold change >1.5.

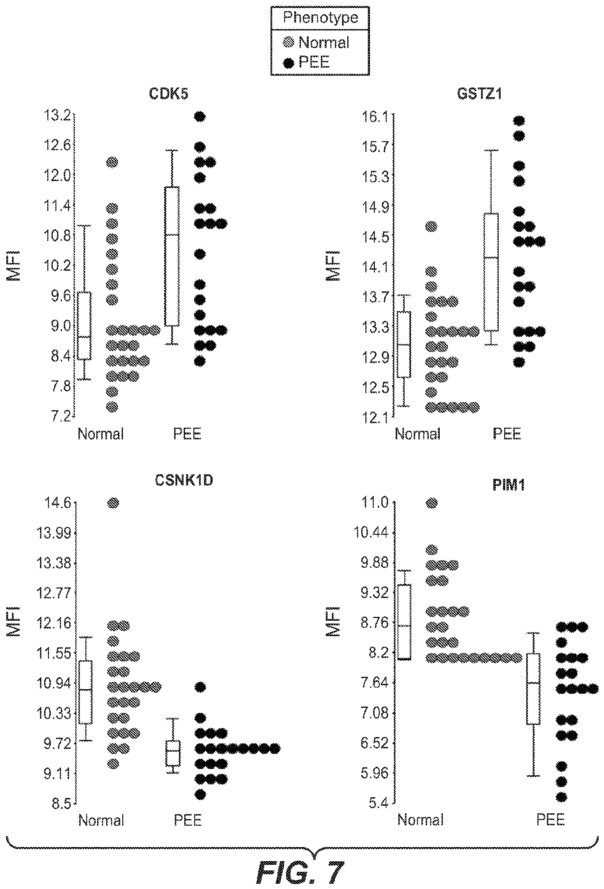

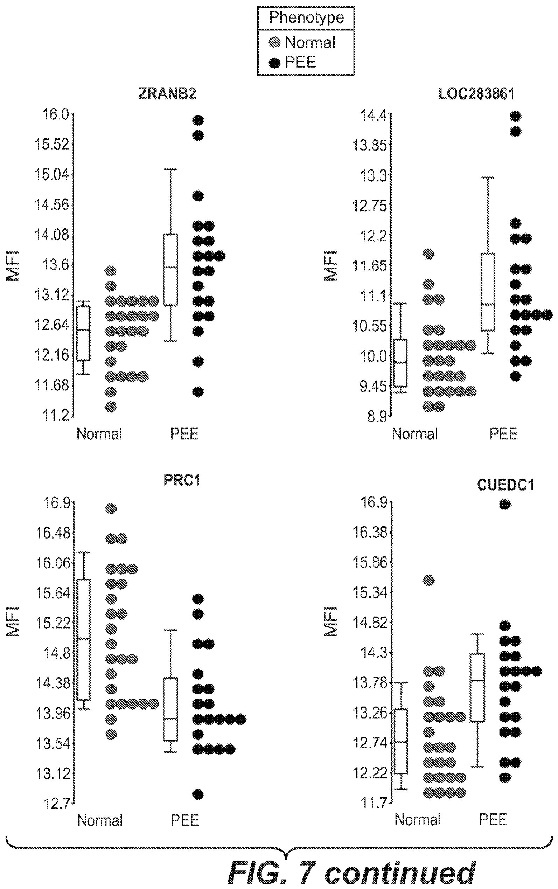

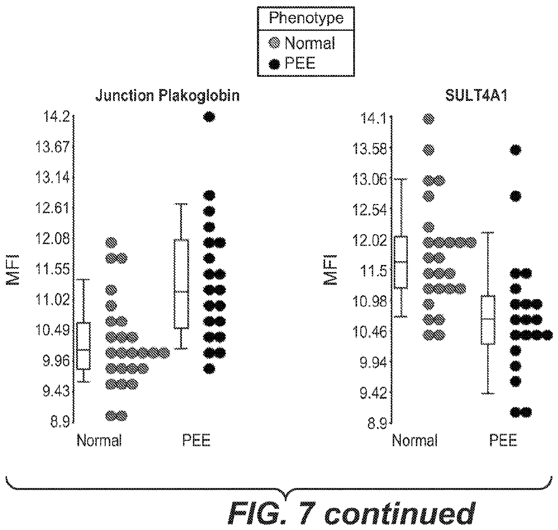

Figure 7 shows scatter plots of the individual mean fluorescent intensities (MFI) counts for each patient for the most significant 10 autoantibodies from the set of 75 PEE autoantibodies which were significantly different (qFDR<0.05; fold change >1.5) in sera taken from pregnant women with and without preeclampsia; displayed are the mean MFI counts plus SEM of each autoantibody in serum samples from 25 healthy pregnant women and 20 pregnant women with PEE.

### DETAILED DESCRIPTION OF THE INVENTION

The invention described herein provides, *inter alia,* methods, compositions, and kits useful for identifying the risk of developing preeclampsia (PEE), predicting the onset of PEE, monitoring the progression of PEE, monitoring the regression of PEE, identifying a sub-population of patients who should be treated for PEE or continue to be treated for PEE, assessing efficacy of treatment for PEE in pregnant women, and/or identifying a sub-population of patients who should be monitored for PEE symptoms. As further detailed below, particular peptide, protein and autoantibody biomarkers have been identified that may be utilized to accurately identify pregnant women during early to mid-pregnancy that may later develop PEE. Such markers may allow the diagnostic distinction between PEE and other conditions that exhibit similar symptoms. Early identification of subjects at greater risk for PEE would be of considerable value to help to save both the baby and the mother's lives and improve their welfare.

### Definitions

For purposes of interpreting this specification, the following definitions will apply and whenever appropriate, terms used in the singular will also include the plural and vice versa.

As used herein, "preeclampsia" or "PEE" refers to a condition when a pregnant woman develops high blood pressure (relative to her blood pressure before pregnancy) and/or protein in the urine during pregnancy. In many cases, the high blood pressure and/or protein in the urine occurs after the 20th week (late 2nd or 3rd trimester) of pregnancy. Symptoms of preeclampsia can include, but are not limited to, high blood pressure (hypertension), excess protein in urine (proteinuria), headaches, changes in vision (including temporary loss of vision, blurred vision or light sensitivity), abdominal pain (such as upper abdominal pain, e.g., under the ribs on the right side), nausea, vomiting, dizziness, decreased urine output, sudden weight gain, visual disturbances (e.g., oversensitivity to light, blurred vision, seeing flashing spots or auras), confusion, anxiety, and/or shortness of breath. In some cases, serious signs of PEE include, but are not limited to: high blood pressure, risk of brain injury, impaired kidney and liver function, blood clotting problems, pulmonary edema, and/or seizures. Other symptoms of PEE are described herein and known to one of skill in the art, including treating physicians.

An individual "at risk" of developing PEE may or may not have detectable disease or symptoms of disease, and may or may not have displayed detectable disease or symptoms of disease prior to the treatment methods described herein. "At risk" denotes that a individual has one or more risk factors, which are measurable parameters that correlate with development of PEE, as described herein and known in the art. A subject having one or more of these risk factors has a higher probability of developing PEE than a subject without one or more of these risk factor(s). For example, in some embodiments, a subject "at risk" of developing PEE shows a change in the level of expression of one or more PEE proteins as shown in Table 1 or Table 2.

An "individual" can be a "patient." A "patient," refers to an "individual" who is under the care of a treating physician. In one embodiment, the patient is a female. In another embodiment, the patient is a female who has not been diagnosed with PEE. In yet other embodiments, the patient is a female who has been diagnosed with PEE but has not had any treatment to address the PEE.

A "patient sub-population," and grammatical variations thereof, as used herein, refers to a patient subset characterized as having one or more distinctive measurable and/or identifiable characteristics that distinguishes the patient subset from others in the broader disease category to which it belongs. Such characteristics include having a PEE protein and/or autoantibody profile described herein as being characteristic of being at risk for developing PEE, optionally in combination with any of the symptoms described herein and known to one of skill in the art, including treating physicians.

The term "biological sample," as used herein, refers to a composition that is obtained or derived from an individual that contains a cellular and/or other molecular entity that is to be characterized and/or identified, for example based on physical, biochemical, chemical and/or physiological characteristics. In some embodiments, the biological sample is blood, serum, biological fluid or tissue from the mother. In other embodiments, the biological sample contains some material from the baby.

"Predicting" and "prediction" as used herein does not mean that the event will happen with 100% certainty. Instead it is intended to mean the event will more likely than not happen. Acts taken to "predict" or "make a prediction" can include the determination of the likelihood that an event will be more likely than not to happen. Assessment of multiple factors described herein can be used to make such a determination or prediction.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of PEE protein analysis or PEE autoantibody analysis performed on biological samples from an individual, one may use the results to determine whether a specific therapeutic regimen should be performed for that individual.

The term "diagnosis" is used herein to refer to the identification or classification of a medical or pathological state, disease or condition. For example, "diagnosis" may refer to identification of PEE, "Diagnosis" may also refer to the classification of a severity of PEE. Diagnosis of PEE may be made according to any protocol that one of skill of art (e.g., obstetrician) would use, for example, those set forth in "Pre-eclampsia: Etiology and Clinical Practice" (F. Lyall, Ed. and M. Belfort, Ed.), Cambridge University Press (2007) and/or "Chesley's Hypertensive Disorders in Pregnancy, 3rd edition (M. Lindheimer, J. Roberts, F.G. Cunningham, Eds.), Elsevier (2009).

The term "aiding diagnosis" is used herein to refer to methods that assist in making a clinical determination regarding the presence, degree or other nature, of a particular type of symptom or condition of PEE. For example, a method of aiding diagnosis of PEE can include measuring the amount or detecting the presence or absence of one or more PEE proteins and/or PEE autoantibodies in a biological sample from an individual.

The term "prognosis" is used herein to refer to the prediction of the likelihood of the development of PEE (including recurrence of PEE). The predictive methods of the invention can be used clinically to make treatment decisions by choosing the most appropriate treatment modalities for any particular patient. The predictive methods of the present invention are valuable tools in predicting if and/or aiding in the diagnosis as to whether a patient is likely to develop PEE, have recurrence of PEE, and/or worsening of PEE symptoms.

"Treatment" refers to clinical intervention in an attempt to alter the natural course of the individual and can be performed before, during, or after the course of clinical diagnosis or prognosis. Desirable effects of treatment include preventing the occurrence or recurrence of PEE or a condition or symptom thereof, alleviating a condition or symptom of PEE, diminishing any direct or indirect pathological consequences of PEE, decreasing the rate of PEE progression or severity, and/or ameliorating or palliating the PEE. In some embodiments, methods and compositions of the invention are used on patient sub-populations identified to be at risk of developing PEE. In some cases, the methods and compositions of the invention are useful in attempts to delay development of PEE.

It is understood that aspects and embodiments of the invention described herein include "consisting" and/or "consisting essentially of" aspects and embodiments.

As used herein, the term "peptide" may be used to refer to a natural or synthetic molecule comprising two or more amino acids linked by the carboxyl group of one amino acid to the alpha amino group of another. A peptide of the present invention is not limited by length, and thus "peptide" can be part of a longer polypeptide and/or of a protein or can refer to the longer polypeptide/protein itself. The term peptide can be used interchangeably with protein and/or polypeptide.

As used herein, the term "detect" refers to the quantitative measurement of undetectable, low, normal, or high serum concentrations of one or more biomarkers such as, for example, proteins, peptides and other biological molecules.

As used herein, the terms "quantify" and "quantification" may be used interchangeably, and refer to a process of determining the quantity or abundance of a substance in a sample (e.g., a biomarker), whether relative or absolute.

Reference to "about" a value or parameter herein includes (and describes) embodiments that are directed to that value or parameter per se. For example, description referring to "about X" includes description of "X." The term "about" is used to provide flexibility to a numerical range endpoint by providing that a given value may be "a little above" or "a little below" the endpoint without affecting the desired result. Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited.

As used in the specification and the appended claims, the singular forms "a," "an" and "the" include plural referents unless the context clearly indicates otherwise.

"Optional" or "optionally" means that the subsequently described event or circumstance can or cannot occur, and that the description includes instances where the event or circumstance occurs and instances where it does not.

### General Techniques

Unless defined otherwise, technical and scientific terms used herein have the same meaning as commonly understood by one of ordinary skill in the art to which this invention belongs.

The practice of the present invention will employ, unless otherwise indicated, conventional techniques of protein biology, protein chemistry, molecular biology (including recombinant techniques), microbiology, cell biology, biochemistry, and immunology, which are within the skill of the art. Such techniques are explained fully in the literature, such as, "Pre-eclampsia: Etiology and Clinical Practice" (F. Lyall, Ed. and M. Belfort, Ed.), Cambridge University Press (2007); "Chesley's Hypertensive Disorders in Pregnancy, 3rd edition (M. Lindheimer, J. Roberts, F.G. Cunningham, Eds.), Elsevier (2009);" "Molecular Cloning: A Laboratory Manual", second edition (Sambrook et al., 1989); "Oligonucleotide Synthesis" (M. J. Gait, ed., 1984); "Current Protocols in Molecular Biology" (F. M. Ausubel et al., eds., 1987, periodic updates); "PCR: The Polymerase Chain Reaction", (Mullis et al., eds., 1994); and Singleton et al., Dictionary of Microbiology and Molecular Biology 2nd ed., J. Wiley & Sons (New York, N.Y. 1994).

### Collection of Biological Samples from Pregnant Women

Typically, a biological sample is collected from the pregnant woman. Any type of biological sample may be collected, including but not limited to serum, plasma, blood, urine, mucus, saliva, cerebrospinal fluid, amniotic fluid, synovial fluid, cervical vaginal fluid, lavage fluid, placenta, other surrounding tissues, tissues, and combinations thereof. In one embodiment, the biological sample collected from the pregnant woman contains only non-fetal tissue. In another embodiment, some fetal tissue is collected along with the maternal non-fetal tissue.

Testing of women for PEE using the methods described herein may occur at any time during pregnancy when biomarkers such as proteins, peptides, and autoantibodies indicative of PEE are quantifiable. Biomarkers may be collected and tested for during the first, second, or third trimesters of pregnancy. In one embodiment biomarkers may be collected and tested for at from about 4 weeks to about 6 weeks gestation; at from about 6 weeks to about 8 weeks gestation; at from about 8 weeks to about 10 weeks gestation; at from about 10 weeks to about 12 weeks gestation; at from about12 weeks to about 14 weeks gestation; at from about 14 weeks to about 16 weeks gestation; at from about 16 weeks to about 18 weeks gestation; at from about 18 weeks to about 20 weeks gestation; at from about 20 weeks to about 22 weeks gestation; at from about 22 weeks to about 24 weeks gestation; at from about 24 weeks to about 26 weeks gestation; at from about 26 weeks to about 28 weeks gestation; at from about 28 weeks to about 30 weeks gestation; or at from about 30 weeks and beyond. These ranges should not be seen as limiting, as such testing may be performed at any point during pregnancy. Rather these ranges are provided to demonstrate periods of the gestational cycle where such testing is most likely to occur in a majority of pregnant women.

### Identification of PEE Proteins and Peptides and Testing of Biological Samples

Methods for testing a pregnant woman for PEE may include detecting the difference in the concentration, expression, intracellular translocation, or activity of one or more peptides, proteins, and/or autoantibodies associated with PEE present in a biological sample as compared to a healthy pregnant woman who does not develop PEE. Various systems and methods, as further described herein, can be used to identify, characterize, and quantify the peptides, proteins, or autoantibodies. Non-limiting systems and methods are provided herein.

In one embodiment, mass spectrometry can be used to identify peptides or proteins that are differentially expressed between pregnant women with PEE or suspected of being at risk for developing PEE and pregnant healthy women. In such an embodiment, comparing multiple mass spectra from different biological samples, locating mass ions that are quantitatively different after using approaches to compensate for non-biological variability, isolating, and characterizing the protein or peptide biomarker of interest can be used herein.

In another embodiment, capillary liquid chromatography can be used to identify peptides or proteins that are differentially expressed between pregnant women with PEE or suspected of being at risk for developing PEE and pregnant healthy women. Those of skill in the art will appreciate that other techniques can be used to identify PEE proteins and/or peptides. The features of PEE proteins and/or peptides that enable them to be used as diagnostic markers for PEE is described *infra.*

The proteins or peptides that are differentially expressed can be analyzed by one- or multiple way-analysis of variance (ANOVA) after quantile normalization to identify differentially expressed proteins between PEE and healthy pregnancies. See, for example, Figure 1 and Table 1. In one embodiment, a protein upregulated with a fold change greater than 1.5, or a protein downregulated with a fold change greater than 1.5, with a p-value with false discovery rate of less than 0.05 can be considered to be significant and utilized to build prediction models. In another embodiment, a protein upregulated with a fold change greater than 2, or a protein downregulated with a fold change greater than 2, with a p-value with false discovery rate of less than 0.05 can be considered to be significant and utilized to build prediction models. Various types of software can be used for statistical analysis. One example of such software is Partek Genomics Suite. The proteins or peptides can be subjected to statistical analysis to select a robust model for detection and/or prediction of PEE among the following classification models: K-nearest neighbor, nearest centroid, discriminate analysis, support vector machine, partial least squares, diagonal discriminant analysis, random forest and logistic regression. As further detailed in the Examples, multiple models can yield a receiver operator characteristic curve (ROC) with an area under the curve (AUC) of >0.9, with as few as 3 proteins. Typically, the area under the ROC curve is a measure of testing accuracy that takes into account both measures of sensitivity and specificity. An AUC of 1.0 means that there is 100% accuracy in that cohort. In various embodiments, the area under the ROC curve can be a statistical measurement of the accuracy of detection of PEE, the accuracy in the diagnosis of PEE, the accuracy in the prediction of PEE, the accuracy in the prediction of the onset of PEE, and the like.

Using this methodology, exemplary peptides and/or proteins that were found to be associated with PEE are described in Example 1 and Table 1. These peptides and/or proteins can interchangeably be referred to as "PEE proteins" or "PEE peptides" or "PEE polypeptides" and are useful in the diagnosis, aiding of diagnosis, and/or treatment of PEE. These PEE proteins can also be used to identify patient sub-populations for treatment for PEE.

### Testing of Biological Samples and Identification of PEE Autoantibodies

Biological samples taken from pregnant women can be used to identify autoantibodies that can be used to assess whether a pregnant woman has or will develop PEE (i.e., PEE autoantibodies). Various techniques of measuring autoantibodies are known to one of skill in the art. One non-limiting method is to use a ProtoArray® human protein microarray V5 (microarray) (Invitrogen, Carlsbad, CA) to measure a variety of autoantibodies in biological samples from healthy pregnant women and PEE pregnant women. Relative fluorescent units representing the abundance of each autoantibody in the serum of healthy and PEE pregnancies can be uploaded to and analyzed using any type of statistical software, such as Partek's Genomics Suite (Partek Inc, St. Louis, MO). The data can be quantile normalized followed by one- or multiple way analysis of variance (ANOVA) to identify differentially expressed autoantibodies between PEE and healthy pregnancies.

Exemplary differentially expressed autoantibodies between PEE and healthy pregnancies are shown in Table 4, Table 5, and in Figures 5-7. In one embodiment, an autoantibody upregulated with a fold change greater than 1.5, or an autoantibody downregulated with a fold change greater than 1.5, with a p-value with false discovery rate of less than 0.05 can be considered significant and utilized to build prediction models. In one embodiment, at least about 8 antibodies can provide explanation of 70% of the variation between PEE and healthy pregnancy samples. In another embodiment about 15 antibodies can provide explanation of all of the 95% of the differences between the samples. The PEE autoantibodies disclosed here in can be used for various methods of assessing risk of developing PEE, monitoring the development of PEE and selecting patients for treatment as well as other uses described herein.

### Binding Agents and Methods of Using PEE Proteins and/or PEE Autoantibodies for Detecting PEE or Diagnosing the Risk of Developing Preeclampsia

Binding agents of the invention may be used to identify proteins, peptides, and/or autoantibodies present in the biological samples taken from a pregnant woman suspected of being at risk for developing PEE, already suffering from PEE, or from a healthy pregnant woman. The binding agent can be one or more proteins, one or more peptides, one or more antibodies, one or more nucleic acids, or one or more nucleoproteins. The binding agent can comprise a plurality of binding sites for proteins, peptides, and autoantibodies. In one embodiment, the binding agent can be used to identify a protein or peptide that would predict if a pregnant woman will develop PEE or has PEE, or is recovering from PEE. In such cases, the binding agent can be used to aid in the diagnosis of PEE or PEE status.

Binding agents of the invention may be labeled or modified in a manner know to those with skill in the art. For example binding agents may comprise a label. The label may include, but is not limited to a fluorescent label, an immunolabel, a magnetic label, a DNA label, a small molecule label, or a radio label.

A protein binding agent may be labeled or modified in some manner. For example protein binding agents may comprise a label. The label may include, but is not limited to a fluorescent label, an immunolabel, a magnetic label, a DNA label, a small molecule label, or a radio label.

A peptide binding agent may be labeled or modified in some manner. For example peptide binding agents may comprise a label. The label may include, but is not limited to a fluorescent label, an immunolabel, a magnetic label, a DNA label, a small molecule label, or a radio label.

A nucleic acid binding agent may be labeled or modified in some manner. For example nucleic acid binding agents may comprise a label. The label may include, but is not limited to a fluorescent label, an immunolabel, a magnetic label, a DNA label, a small molecule label, or a radio label.

A nucleoprotein binding agent may be labeled or modified in some manner. For example nucleoprotein binding agents may comprise a label. The label may include, but is not limited to a fluorescent label, an immunolabel, a magnetic label, a DNA label, a small molecule label, or a radio label.

The binding agent can bind one or more proteins, peptides, or autoantibodies with a dissociation constant (K_{d}) of 10⁻¹⁵ M, 10⁻¹⁴ M, 10⁻¹³ M, 10⁻¹² M, 10⁻¹¹ M, 10⁻¹⁰ M, 10⁻⁹ M, 10⁻⁸ M, 10⁻⁷ M, 10⁻⁶ M, 10⁻⁵ M, 10⁻⁴ M, 10⁻³ M, or 10⁻² M. In certain embodiments, the binding agent binds the one or more proteins, peptides, or autoantibodies with a K_{d} range of 10⁻¹² M to 10⁻⁵ M, 10⁻¹⁰ M to 10⁻⁵ M, 10⁻⁸ M to 10⁻⁵ M, 10⁻⁷ M to 10⁻⁵ M, 10⁻¹⁰ M to 10⁻⁸ M, 10⁻⁹ M to 10⁻⁷ M, or 10⁻⁸ M to 10⁻⁶ M.

In one specific embodiment the binding agent can bind 1 protein, peptide, and/or autoantibody. In another embodiment the binding agent can bind 2 proteins, peptides, and/or autoantibodies. In yet another embodiment, the binding agent can bind 3 proteins, peptides, and/or autoantibodies. In related embodiments, the binding agent can bind 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, proteins, peptides, and/or autoantibodies, up to a maximum of 30, 40, 50, 60, 70, 80, 90, or 100 proteins, peptides, and/or autoantibodies. In one specific embodiment the binding agent can bind a maximum of 48 PEE proteins and/or peptides. In another specific embodiment the binding agent can bind any combination of 2, 3, 4, 5, 6, 7, 8, 9, 10 or more combinations of proteins, peptides and antibodies.

The PEE proteins and PEE autoantibodies as described herein can be used to diagnose or aid in the diagnosis of individuals who are at risk of developing PEE. The PEE proteins and PEE autoantibodies can also be used to identify the risk of developing preeclampsia, predict the onset of PEE, monitor the progression of PEE, monitor the regression of PEE, identify a sub-population of patients who should be treated for PEE or continue to be treated for PEE, assess efficacy of treatment for PEE in pregnant women, and/or identify a sub-population of patients who should be monitored for PEE symptoms.

In one embodiment, the binding agents of the invention are selected from antibodies, autoantibodies, peptides, polypeptides, oligonucleotides, small molecules, and the like. In a specific embodiment, binding agents of the invention comprise a fluorescent label, or a fluorescent immunolabel. In another specific embodiment, the binding agents of the invention comprise a magnetic label or magnetic immunolabel. In another specific embodiment, the binding agents of the invention comprise a radio label or magnetic radio label. In yet another embodiment, the binding agents are labeled with a oligonucleotide or a small molecule.

In one embodiment a binding agent comprises an immune assay. An immunoassay can be used to detect, identify and/or quantify proteins or peptides present in the biological samples taken from a pregnant woman suspected of being at risk for developing PEE and a healthy pregnant woman. In certain embodiments, the immunoassay can be an enzyme-linked immunosorbant assay (ELISA). Any immunoassay used herein can incorporate fluorescent, magnetic, or radio immunolabels.

In another embodiment, a binding agent comprises a diagnostic array. A diagnostic array can be used to detect, identify and/or quantify proteins, peptides, or autoantibodies present in the biological samples taken from a pregnant woman suspected of being at risk for developing PEE and a healthy pregnant woman. The array can include a protein or antibody-coated substrate comprising a plurality of discrete, known regions on the substrate. The arrays can comprise particles, nanoparticles, beads, nanobeads, or other solid surfaces which can be porous or non-porous, and can range in size. In one embodiment, the diagnostic array does not comprise fluorescent particles. In another embodiment, the diagnostic array comprises fluorescent particles. In one embodiment, the diagnostic array does not comprise magnetic particles. In another embodiment, the diagnostic array comprises magnetic particles.

In a related embodiment, the binding agents of the invention comprise particles, nanoparticles, beads, nanobeads. In one embodiment, the nanoparticles, beads, or nanobeads are fluorescently labeled. In another embodiment, the nanoparticles, beads, or nanobeads are magnetically labeled. In another embodiment, the nanoparticles, beads, or nanobeads are radio labeled. In yet another embodiment, the nanoparticles, beads, or nanobeads are labeled with a oligonucleotide or a small molecule.

In another embodiment, a binding agent comprises a magnetic-based protein assay component and/or nanotags. In such an embodiment, a magnetic multiplex protein assay is used to detect, identify, and/or quantify proteins or peptides present in a biological sample with the use of magnetic nanotags. (Osterfeld et al., "Multiplex Protein Assays Based on Real-Time Magnetic Nanotag Sensing," PNAS, 105, 20637-20640 (published online Dec.12, 2008) For example, a MagArray protein chip can be utilized for the diagnostic array. In this embodiment, protein or peptide detection is used carried out in three steps. First, probes on the surface specifically bind to proteins or peptides in the sample. Second, nanotag-labeled antibodies bind to the bound proteins or peptides, forming sandwich-like structures. Finally, an external magnetic field is applied to the chip and the stray magnetic field produced by the nanotags is measured electrically to determine the presence of the target molecule in the sample.

In a related embodiment, the binding agents of the invention comprise nanotags. In one embodiment, the nanotags are fluorescently labeled. In another embodiment, the nanotags are magnetically labeled. In another embodiment, the nanotags are radio labeled. In yet another embodiment, the nanotags are labelled with a oligonucleotide or a small molecule.

In another embodiment, carboxyl bead sets can be used to measure proteins, peptides of interest. Here, any protein or peptide can be covalently attached to a stable microbead surface followed by fluorescent labeling and fluorescence intensity measurement. The VeraCode Technology by Illumina (Illumina Inc., Hayward, CA) allows one to perform up to 48 immunoassays in varying combinations in a single reaction in a standard 96-well microplate.

In yet another embodiment proteins, peptides and autoantibodies can be measured by electrochemiluminesence ELISA. The multiplexed electrochemiluminesence ELISA platform by Meso Scale Discovery (MSD, Gaithersburg, MD) is a high throughput multiplexed ELISA, custom designable, with the capability to simultaneously measure up to several analytes in the same well.

In another embodiment, functional protein-based assays can be used to detect differences in activity, binding, intracellular translocation, or post-translational processing of a protein, peptide, or autoantibody biomarker of interest. Such assays include competitive binding assays, western blot immunoblot assays, liposome immunoassays, and the like. In one specific embodiment, and by way of example only, an assay such as Invitrogen's ProtoArray® Microarray can be used to detect protein-protein interactions of interest. This array allows for profiling a biological sample such as serum or urine from a pregnant woman suspected for being at risk for PEE and can be used for identifying biologically relevant protein kinase substrates, small molecule binding partners, ubiquitin ligase substrates, and proteins interactors of antibodies. In one embodiment, spectral imaging can be used to detect differences in the characteristics of proteins.

The PEE proteins and/or PEE autoantibodies can be detected by a binding agent with the functional parameter as described in the sections above. In other embodiments, the binding agent can be used to quantify PEE proteins, peptides and/or autoantibodies. This may be useful to predict the onset of PEE, the risk of developing PEE, to diagnose PEE, or to determine the severity of PEE symptoms.

One benefit of using the PEE proteins and/or PEE autoantibodies as disclosed herein is that determination of the risk of developing preeclampsia can be done with a high level of accuracy. Accuracy can be portrayed by sensitivity (the accuracy of the preeclampsia positive patients correctly identified) and by specificity (the accuracy of the preeclampsia negative patients correctly identified); positive predictive value (PPV) and negative predictive value (NPV) respectively.

In the embodiments provided herein, determination of the risk of developing PEE using the PEE proteins and/or PEE autoantibodies or a combination of both peptides/proteins and autoantibodies for a pregnant woman suspected to be at risk for developing PEE is highly accurate for the detection or prediction of PEE. In the embodiments provided herein, the methods provide at least 60%, at least 65%, at least 70%, at least 75%, at least 80%, at least 85%, at least 90%, at least 95%, at least 96%, at least 97%, at least 98%, at least 99%, or 100% accuracy. Furthermore, in the embodiments provided herein, the methods provide at least 60%, at least 65%, at least 70%, at least 75%, at least 80%, at least 85%, at least 90%, at least 95%, at least 96%, at least 97%, at least 98%, at least 99%, or 100% accuracy for the detection, or prediction of PEE.

In the embodiments provided herein, determination of the risk of developing PEE using the PEE proteins and/or PEE autoantibodies or a combination of both peptides/proteins and autoantibodies for a pregnant woman suspected to be at risk for developing PEE is highly sensitive for the detection or prediction of PEE. In the embodiments provided herein, the methods provide at least 60%, at least 65%, at least 70%, at least 75%, at least 80%, at least 85%, at least 90%, at least 95%, at least 96%, at least 97%, at least 98%, at least 99%, or 100% sensitivity. Furthermore, in the embodiments provided herein, the methods provide at least 60%, at least 65%, at least 70%, at least 75%, at least 80%, at least 85%, at least 90%, at least 95%, at least 96%, at least 97%, at least 98%, at least 99%, or 100% sensitivity for the detection, or prediction of PEE.

Furthermore in the embodiments provided herein, analysis of protein, peptide, and/or autoantibody biomarkers from a pregnant woman suspected to be at risk for developing PEE is highly specific for the detection or prediction of PEE. In the embodiments provided herein, the methods provide at least 60%, at least 65%, at least 70%, at least 75%, at least 80%, at least 85%, at least 90%, at least 95%, at least 96%, at least 97%, at least 98%, at least 99%, or 100% specificity. Furthermore, in the embodiments provided herein, the methods provide at least 60%, at least 65%, at least 70%, at least 75%, at least 80%, at least 85%, at least 90%, at least 95%, at least 96%, at least 97%, at least 98%, at least 99%, or 100% specificity for the detection, or prediction of PEE.

Moreover, in the embodiments provided herein, analysis of protein, peptide, and/or autoantibody biomarkers from a pregnant woman suspected to be at risk for developing PEE has a positive predictive value (PPV; the proportion of positive test results that are true positives/correct diagnoses) for the detection or prediction of PEE. In the embodiments provided herein, the methods provide at least 60%, at least 65%, at least 70%, at least 75%, at least 80%, at least 85%, at least 90%, at least 95%, at least 96%, at least 97%, at least 98%, at least 99%, or 100% PPV for the detection or prediction of PEE. Also, in the embodiments provided herein, analysis of biomarkers from a pregnant woman suspected to be at risk for developing PEE has a negative predictive value (NPV; the proportion of subjects with a negative test result who are correctly diagnosed) for the detection or prediction of PEE. In the embodiments provided herein, the methods provide at least 60%, at least 65%, at least 70%, at least 75%, at least 80%, at least 85%, at least 90%, at least 95%, at least 96%, at least 97%, at least 98%, at least 99%, or 100% NPV, for the detection or prediction of PEE.

In the embodiments provided herein, the analysis of biomarkers from a pregnant woman suspected to be at risk for developing PEE provides an area under the curve (AUC), which is a statistical measurement of the probability of the detection of PEE, or a statistical measurement of the probability for predicting the development of PEE. In the embodiments provided herein, the methods provide an AUC of at least 0.80, at least 0.81, at least 0.82, at least 0.83, at least 0.84, at least 0.85, at least 0.86, at least 0.87, at least 0.88, at least 0.89, at least 0.90, at least 0.91, at least 0.92, at least 0.93, at least 0.94, at least 0.95, at least 0.96, at least 0.97, at least 0.98, at least 0.99, and 1.0 for the detection of PEE or for predicting the development of PEE.

The analysis of biological samples taken from either a pregnant woman suspected to be at risk for developing PEE or from a healthy pregnant woman include testing for only 1, testing for combinations of 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more proteins, peptides, and/or autoantibodies, up to a maximum of 30, 40, 50, 60, 70, 80, 90, or 100 PEE proteins, PEE peptides, and/or PEE autoantibodies disclosed herein.

In one embodiment, the analysis of biomarkers from a pregnant woman suspected to be at risk for developing PEE comprises detecting an increase or decrease in at least one protein selected from Table 1. In such an embodiment, the risk for developing PEE can comprise a change in the protein concentration of IGLC1, C8G, C2, APOA4, TF, SERPIND1, CIS, THBS1, APCS, CRP, IGFBP3, IGKC V-IV, ITIH1, CLU, APOC2, PROS1, PAPPA, AFM, VWF, PRG2, APOB, FCN3, C8A, IGHG1, CD14, IGHG3, HPX, IGHG1, IGHG1, CLEC3B, APOE, PSG9, CP, GSN, FN1, FBLN1, KRT86, A2M, APOB, F5, ITIH4, hCG, KRT31, APOL1, HP, MBL2, C4BPB, and/or F13B. In another embodiment, the analysis of biomarkers from a pregnant woman suspected to be at risk for developing PEE comprises a change in at least one protein selected from Table 2. In such an embodiment, the risk for developing PEE can comprise a change in the protein concentration of APCS, THBS1, PRG2, ITIH4, GSN, HPX, F5, and/or TF.

In one embodiment, pregnant women who are at risk for developing PEE can be identified with a high level of accuracy (e.g., at least about 90%, 95%, 99%, or 100%) using 3 proteins. In one embodiment, the 3 proteins are selected from Table 1. In another embodiment, any 3 proteins are selected from the group consisting of APCS, THBS1, PRG2, ITIH4, GSN, HPX, F5, TF, APOB, A2M, C4BPB, FN1, PAPPA, and VWF. In a related embodiment, a combination of 3 proteins is selected from the combinations presented in Table 3.

In another embodiment, the analysis can include testing for up to any one or combination (of 2 or more, or of 3 or more) of the 48 PEE proteins as disclosed in Table 1; or any one or combination (of 2 or more, or 3 more more) of the 8 proteins as disclosed in Table 2. Combinations of the 48 PEE proteins of Table 1 can provide a minimal set of proteins for differentiating the risk of developing PEE from healthy pregnancy. Table 3 provides exemplary combinations of 3 proteins that can be used for detecting the risk of developing PEE in a pregnant woman. In any of these embodiments, the testing can optionally can be done with one or more PEE autoantibodies further disclosed herein, *infra.*

In some embodiments, a minimum set of 3 proteins (the identities can vary for the combinations of 3 proteins) can give an AUC of > 0.96 for separation of women suspected to be at risk for developing PEE from women who will undergo a normal pregnancy. In one embodiment, any combination of 3 proteins can be selected from APCS, THBS1, PRG2, ITIH4, GSN, HPX, F5, TF, APOB, A2M, C4BPB, FN1, PAPPA, and VWF. For example, one combination of 3 proteins can be GSN, THBS1 and PRG2. In other embodiments, a minimum set of 2 proteins can give an AUC of > 0.95 for separation of women suspected to be at risk for developing PEE from women who will undergo a normal pregnancy. For example, one combination of 2 proteins can be THBS1 and PRG2.

In the embodiments provided herein, the analysis of biomarkers from a pregnant woman suspected to be at risk for developing PEE comprises a change (increase or decrease) in at least one autoantibody selected from Table 4 or Table 5. Table 4 provides 75 PEE autoantibodies differentially expressed between PEE and healthy pregnancies. Table 5 provides one subset of 10 autoantibodies differentially expressed between PEE and healthy pregnancies. In one embodiment, the risk for developing PEE comprises an increase in the concentration of an autoantibody to RNF111, ACY3, GSTZ1, SCAMPI, KIAA1826, OSBPL1A, NDUFB2, JAK3, PI16, SCP2, SULT4A1, LOC283861, DLX1, KRT33B, PRKD2, BNIP1, U1snRNP68, KLHL29, PSPH, MEIS3, C16orf28, JUP, CTNNA3, CUEDC1, EMILIN1, CCDC53, UBE1DC1, JAK2, CD40, CAPZA2, DSN1, LOC284837, IGHA1, DACT3, CLIP3, TEC, PSMA1, SNURF, CSNK2A1, CSNK1D, or combinations thereof. In another embodiment, the risk for developing PEE comprises a decrease in the concentration of an autoantibody to AKT1, GAGE7B, SUGT1L1, SLC5A2, HMGB1, GPBP1L1, APEX1, RPS28, RPS10, RBMX, RPL39L, C18orf22, TSLP, GPR45, PIM1, RBMS3, IGLV2-14, CCL19, FGFR3, ANKHD1, ACP1, BEX5, GLO1, PIK3CG, MGC16075, PRC1, ZRANB2, HSPA5, CDK5, HOXC8, TNFSF13B, SUPT4H1, ORC6L, FKSG44, or combinations thereof. In one exemplary embodiment, the risk for developing PEE comprises an increase in the concentration of an autoantibody to glutathione transferase zeta 1 (Maleylacetoacetate isomerase, GSTZ1), sulfotransferase family 4A, member 1 (SULT4A1), Junction plakoglobin (JUP), CUE domain containing 1 (CUEDC1), hypothetical protein LOC284837, and combinations thereof. In another exemplary embodiment, the risk for developing PEE comprises a decrease in the concentration of an autoantibody to casein kinase 1, delta (CSNK1D), pim-1 oncogene (PIM1), protein regulator of cytokinesis 1 (PRC1), zinc finger, RAN-binding domain containing 2 (ZRANB2), cyclin-dependent kinase 5 (CDK5), or combinations thereof. CSNK1D, CUEDC1 and ZRANB2, is one exemplary set of 3 autoantibodies that allows for detection of PEE with an accuracy of 93%, with an area under the curve of 0.93. CSNK1D, SULT4A1 and Junction Plakoglobin is another exemplary set of 3 autoantibodies that allows for detection of PEE with an accuracy of 94%, with an area under the curve of 0.94.

In other embodiments, the assessment of a pregnant woman suspected to be at risk for developing PEE involves testing for the combination of a change in at least one protein selected from Table 1 or 2, a change in at least one autoantibody selected from Table 4 or Table 5, and combinations thereof, including combinations of proteins and autoantibodies.

The PEE proteins and/or PEE antibodies of the invention can also be used to identify a patient sub-population of pregnant women who are at risk for preeclampsia for treatment purposes. In some embodiments, this sub-population is monitored for development, progression, or regression of PEE symptoms.

In some embodiments, this sub-population is treated for PEE prior to or at the onset of PEE symptoms. In some embodiments, the treatment is delivery of the baby. In other embodiments, the treatment can be administering to the pregnant women suitable anti-hypertensive medications to lower the blood pressure, corticosteroids and/or anti-convulsive medication. In other embodiments, the treatment is bed rest for the pregnant woman to lower the expectant mother's blood pressure and/or to increase blood flow to the placenta. This sub-population of patients can be monitored for various physiological parameters known to a treating physician at all stages to ensure their safety and their child's safety. In some cases, the monitoring is done to determine if the treatment should be continued or to see if the treatment is efficacious.

Therefore, using the PEE proteins and/or PEE antibodies of the invention and the methodology described herein, one of skill in the art can determine the risk of developing PEE, can determine the onset of PEE, monitor the progression of PEE, monitoring the regression of PEE, identify a sub-population of patients who should be treated for PEE or continue to be treated for PEE, assess efficacy of treatment for PEE in pregnant women, and/or identify a sub-population of patients who should be monitored for PEE symptoms.

### Kits for the Diagnosis, Detection, or Prediction of PEE

The invention further provides for assay kits for the diagnosis, detection and prediction of PEE. A kit comprises reagents for detecting a protein, peptide, or autoantibody or a combination of protein, peptide and autoantibody implicated in PEE, in a biological sample from a pregnant woman. The reagents can comprise binding agents of the invention. The binding agents and reagents found in the kit may be labeled. They may be labeled, for example, with a fluorescent label, a radiolabel, an immunolabel, a magnetic label, a small molecule label, a DNA-based label, and/or any labels known to those in the art. The kit further comprises a composition comprising one or more solid surfaces that contain at least binding agent, capable of specifically binding a protein, peptide, or autoantibody biomarker (or combinations thereof) of interest in the biological sample. The kit also comprises instructions for the use of the assay. In one embodiment the binding agent is capable of binding to a peptide or protein. In another embodiment the binding agent is capable of binding to an autoantibody. In one specific embodiment, the kit comprises a composition comprising one or more solid surfaces comprising binding agents for a PEE protein selected from APCS, THBS1, PRG2, ITIH4, GSN, HPX, F5, TF, APOB, A2M, C4BPB, FN1, PAPPA, and VWF. In another specific embodiment, the kit comprises a composition comprising solid surfaces comprising binding agents for the PEE proteins THBS1 and PRG2. In another specific embodiment, the kit comprises a composition comprising solid surfaces comprising binding agents for the PEE proteins GSN, THBS1 and PRG2. In another specific embodiment, the kit comprises a composition comprising solid surfaces comprising binding agents for the PEE autoantibodies CSNK1D, CUEDC1 and ZRANB2. In another specific embodiment, the kit comprises a composition comprising solid surfaces comprising binding agents for the PEE autoantibodies CSNK1D, SULT4A1 and Junction Plakoglobin.

### Compositions for the Diagnosis. Detection, or Prediction of PEE

The present invention provides for compositions comprising one or more solid surfaces which include binding agents, specific for PEE proteins and PEE autoantibodies. In certain embodiments, the solid surface comprises binding agents for at least 1, 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, proteins, peptides, and/or autoantibodies, up to a maximum of 30, 40, 50, 60, 70, 80, 90, or 100 proteins, peptides, and/or autoantibodies. In one embodiment, a maximum of 48 PEE proteins and/or peptides is measured for risk assessment. In another embodiment, the binding agent(s) can bind a maximum of 48 PEE proteins and/or peptides. In another embodiment, the solid surface can include a binding agent which can bind any combination of 2, 3, 4, 5, 6, 7, 8, 9, 10 or more combinations of PEE proteins, peptides and antibodies. In another specific embodiment, the invention provides a composition which includes one or more solid surfaces comprising binding agents for APCS, THBS1, PRG2, ITIH4, GSN, HPX, F5, TF, APOB, A2M, C4BPB, FN1, PAPPA, and VWF. In another specific embodiment, the invention provides a composition which includes solid surfaces comprising binding agents for the PEE proteins THBS1 and PRG2. In another specific embodiment, the invention provides a composition which includes solid surfaces comprising binding agents for the PEE proteins GSN, THBS1 and PRG2. In another specific embodiment, the invention provides a composition which includes solid surfaces comprising binding agents for the PEE autoantibodies CSNK1D, CUEDC1 and ZRANB2. In another specific embodiment, the invention provides a composition which includes solid surfaces comprising binding agents for the PEE autoantibodies CSNK1D, SULT4A1 and Junction Plakoglobin.

In some embodiments the solid surface comprises a label. In one embodiment the label is an immunolabel, for example, one or more antibodies or autoantibodies comprising labels. In another embodiment, the label is a magnetic label. In another embodiment, the label is a fluorescent label.

The following examples are provided for illustrative purposes. These are intended to show certain aspects and embodiments of the present invention but are not intended to limit the invention in any manner.

### EXAMPLES

### Example 1: Identification of Serum Protein Biomarkers by Mass Spectrometry Based Proteomics

Serum samples from healthy and PEE pregnancies were depleted of the 20 most abundant serum proteins with ProteoPrep20 Plasma Immunodepletion Kit (Sigma-Aldrich, Cat.no PROT20). The eluate from each sample was subsequently subjected to trypsin digestion with a standard trypsin digestion protocol. Tryptic peptides were reconstituted in buffer containing 0.2% formic acid, 2% acetonitrile, and 97.8% water prior to mass spectrometry. The high-performance liquid chromatography (HPLC) utilized was an Eksigent nano2D (Eksigent) with a self-packed 150 uM ID C18, 15 cm column. The electrospray source was a Michrom Advance operated at 600 nL/min on a LTQ Orbitrap Velos (Thermo Fisher). Data acquisition was performed in a data dependent fashion in which the top 12 (Velos) most intense charged peptide ions were selected for MS/MS fragmentation of charge state 2+ and 3+. Data were subsequently extracted with msconvert script into a mzXML format prior to Sorcerer (SAGE-N) analysis with the Sequest algorithm. The International Protein Index (IPI) human database was searched, using a 50 ppm mass window on the precursor ion. The static modification of propionamide on Cys and variable modifications of Met oxidation and Lys acetylation was allowed for. All searches were compiled and displayed in a Scaffold (Proteome Software) interface, which listed identified proteins with cumulative spectral counts for each protein identified.

Protein IDs along with corresponding spectral counts were uploaded to and analyzed with Partek's Genomics Suite (Partek Inc, St. Louis, MO). The data were quantile normalized followed by analysis of variance (ANOVA) to identify differentially expressed proteins between PEE and healthy pregnancies. Figure 1 and Table 1. Proteins with fold change greater than or less than 1.5 with a p-value with false discovery rate of less than 0.05 were considered significant and utilized to build prediction models. The Partek Genomics Suite was utilized to select one robust model for prediction of PEE among the following classification models: K-nearest neighbor, nearest centroid, discriminate analysis, support vector machine, partial least squares, diagonal discriminant analysis, random forest and logistic regression. Multiple models yield a receiver operator characteristic curve (ROC) with an area under the curve (AUC) of 0.95-1.0, with as few as 3 proteins. Any of these models are useful for the diagnosis of PEE and provides a Sensitivity of 0.9 or greater, Specificity of 1.0 (100%), PPV 1.0 (100%), NPV 0.92 (92%) with as few as 2 proteins (for example THSB1 and PRG2) or with 3 proteins (for example: GSN, THBS1, PRG2). Table 2 provides for one set of 8 differentially expressed proteins in pregnant women with PEE as compared to healthy pregnant women. Table 3 provides exemplary combinations of 3 proteins that can be used for detecting the risk of developing PEE in a pregnant woman.

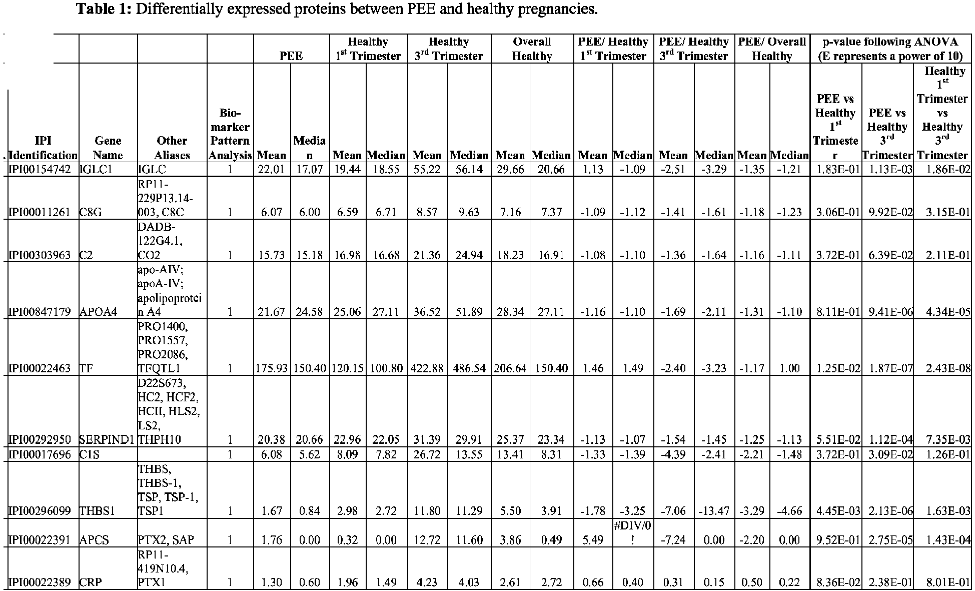

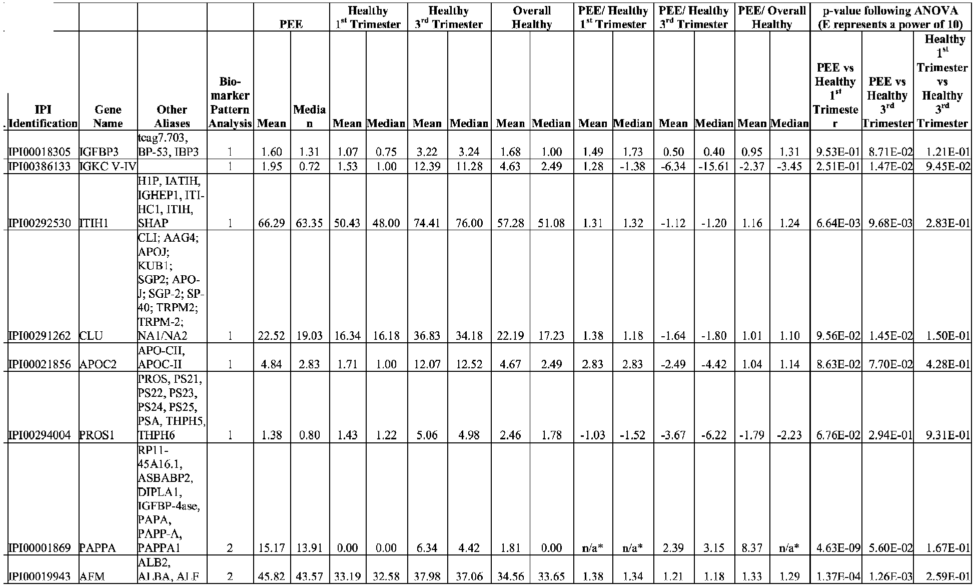

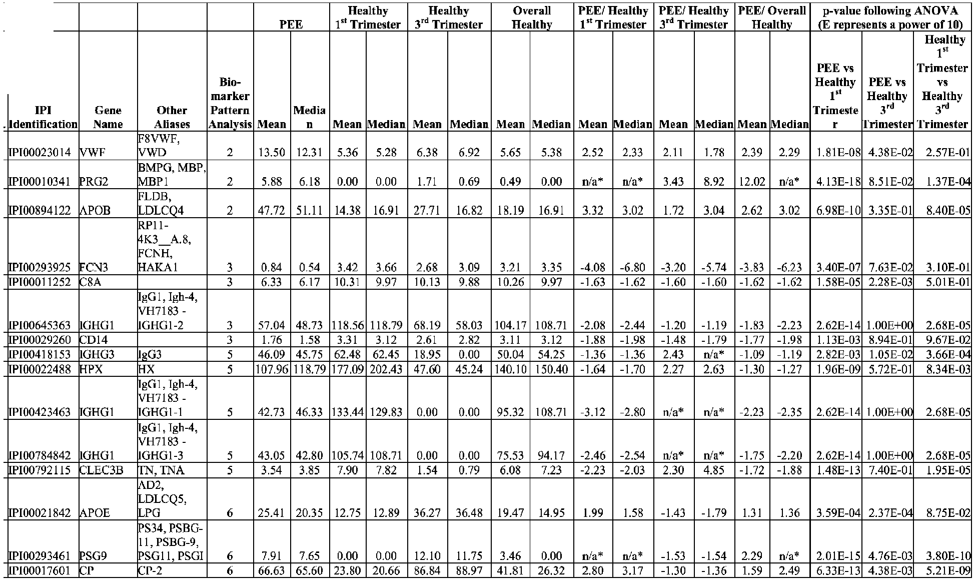

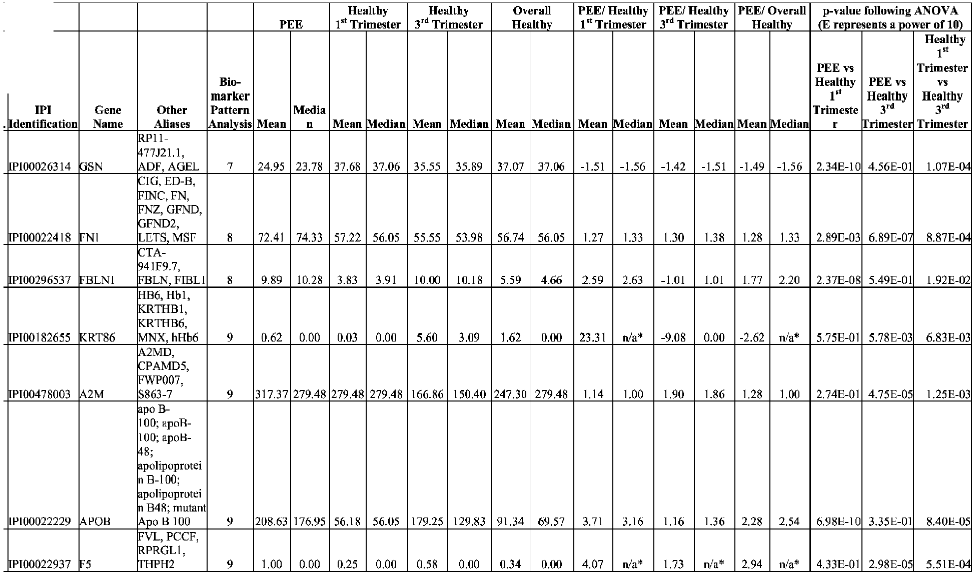

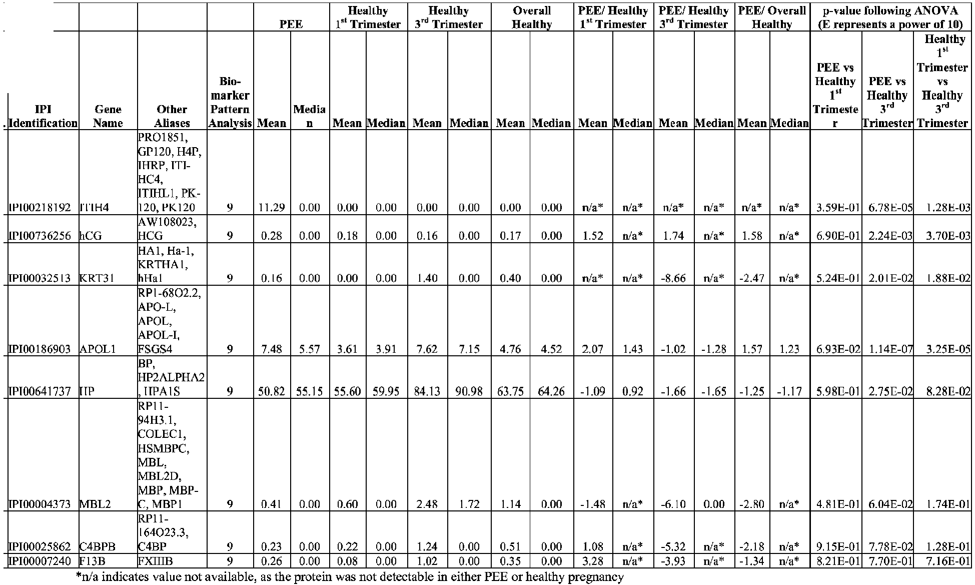

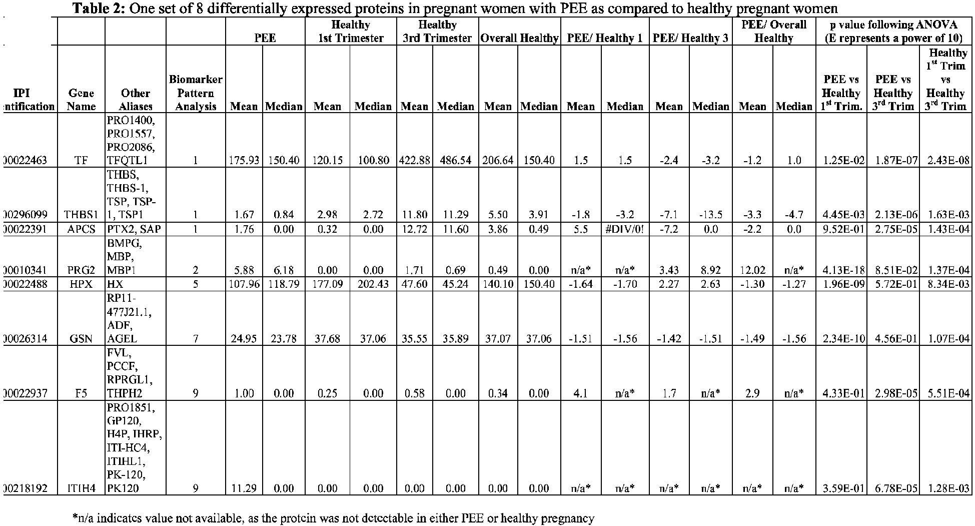

**Table 3 Exemplary combinations of 3 PEE proteins that can be used for diagnosing PEE, detecting the risk of developing PEE, or monitoring the progression or regression of PEE in pregnant women.**

| | **Protein 1** | **Protein 2** | **Protein 3** |
|---|---|---|---|
| **Combination 1** | APCS | THBS1 | PRG2 |
| **Combination 2** | APCS | THBS1 | ITIH4 |
| **Combination 3** | APCS | THBS1 | GSN |
| **Combination 4** | APCS | THBS1 | HPX |
| **Combination 5** | APCS | THBS1 | F5 |
| **Combination 6** | APCS | THBS1 | TF |
| **Combination 7** | APCS | PRG2 | ITIH4 |
| **Combination 8** | APCS | PRG2 | GSN |
| **Combination 9** | APCS | PRG2 | HPX |
| **Combination 10** | APCS | PRG2 | F5 |
| **Combination 11** | APCS | PRG2 | TF |
| **Combination 12** | APCS | ITIH4 | GSN |
| **Combination 13** | APCS | ITIH4 | HPX |
| **Combination 14** | APCS | ITIH4 | F5 |
| **Combination 15** | APCS | ITIH4 | TF |
| **Combination 16** | APCS | GSN | HPX |
| **Combination 17** | APCS | GSN | F5 |
| **Combination 18** | APCS | GSN | TF |
| **Combination 19** | APCS | HPX | F5 |
| **Combination 20** | APCS | HPX | TF |
| **Combination 21** | APCS | F5 | TF |
| **Combination 22** | THBS1 | PRG2 | ITIH4 |
| **Combination 23** | THBS1 | PRG2 | GSN |
| **Combination 24** | THBS1 | PRG2 | HPX |
| **Combination 25** | THBS1 | PRG2 | F5 |
| **Combination 26** | THBS1 | PRG2 | TF |
| **Combination 27** | THBS1 | ITIH4 | GSN |
| **Combination 28** | THBS1 | ITIH4 | HPX |
| **Combination 29** | THBS1 | ITIH4 | F5 |
| **Combination 30** | THBS1 | ITIH4 | TF |
| **Combination 31** | THBS1 | GSN | HPX |
| **Combination 32** | THBS1 | GSN | F5 |
| **Combination 33** | THBS1 | GSN | TF |
| **Combination 34** | THBS1 | HPX | F5 |
| **Combination 35** | THBS1 | HPX | TF |
| **Combination 36** | THBS1 | F5 | TF |
| **Combination 37** | PRG2 | ITIH4 | GSN |
| **Combination 38** | PRG2 | ITIH4 | HPX |
| **Combination 39** | PRG2 | ITIH4 | F5 |
| **Combination 40** | PRG2 | ITIH4 | TF |
| **Combination 41** | PRG2 | GSN | HPX |
| **Combination 42** | PRG2 | GSN | F5 |
| **Combination 43** | PRG2 | GSN | TF |
| **Combination 44** | PRG2 | HPX | F5 |
| **Combination 45** | PRG2 | HPX | TF |
| **Combination 46** | PRG2 | F5 | TF |
| **Combination 47** | ITIH4 | GSN | HPX |
| **Combination 48** | ITIH4 | GSN | F5 |
| **Combination 49** | ITIH4 | GSN | TF |
| **Combination 50** | ITIH4 | HPX | F5 |
| **Combination 51** | ITIH4 | HPX | TF |
| **Combination 52** | ITIH4 | F5 | TF |
| **Combination 53** | GSN | HPX | F5 |
| **Combination 54** | GSN | HPX | TF |
| **Combination 55** | GSN | F5 | TF |
| **Combination 56** | HPX | F5 | TF |

### Example 2: Identification of Antibody-based Biomarkers by High-density Protein Microarrays

The ProtoArray® human protein microarray V5 (microarray) (Invitrogen, Carlsbad, CA) was utilized to identify serum IgG interactions with a panel of antigens. Each microarray contained 9400 separate protein antigens printed in duplicate with N-terminal GST epitopes expressed in Baculovirus and affinity purified under native conditions maintaining their cellular enzymatic activities/native conformations. Each protein was derived from the Ultimate ORF collection (Invitrogen, Carlsbad, CA). Microarrays were stored at -80°C, equilibrated to 4°C for 20 min before use. The microarray slides were blocked with 5.0 mL Blocking Buffer (100 mM Sod. Phosphate pH 7.4, 200 mM NaCl, 0.08% Triton X-100, 25% Glycerol, 20 mM Reduced Glutathione, 1.0 mM DTT, 1% Hammarstein Grade Caesin) with gentle agitation in 4-well trays for 1 hr at 4 °C. After the blocking step, the Blocking Buffer was removed by aspiration and a 5.0 mL diluted serum (1:150) in PBST Buffer (IX PBS, 1% Hammerstein Grade casein, 0.1% Tween 20) was added onto the plate to incubate for 90 min with gentle agitation at 4 °C. In the next step, the serum sample was removed and the plates were washed 5 times with 5.0 mL fresh PBST Buffer with 5 minutes incubations per wash with gentle agitation. A 5.0 mL portion of secondary antibody (anti-human antibody-Alexa Fluor® 647 conjugate) diluted in PBST Buffer was added and the plates were incubated for 90 minutes with gentle agitation at 4 °C. The secondary antibody solution was removed by aspiration. The plates were then washed for 5 times with 5.0 mL fresh PBST Buffer, 5 minutes incubations per wash with gentle agitation. At the end, the slides were dried by centrifugation and scanned using Axon GenePix 4000B Scanner (Molecular Devices, Sunnyvale, CA). The data was acquired by using microarray analysis software GenePix pro 6.0 (Molecular devices, Sunnyvale, CA). The slides were scanned at 635nm with a PMT gain of 600, a laser power of 100% and a focus point of 0 µm. The ".gal" files were obtained from a ProtoArray central portal on the Invitrogen website (www.invitrogen.com/ProtoArray) by submitting the barcode of each protein microarray. Data was extracted from the .gpr files with using Prospector Analyzer® 5.2 (Invitrogen, Carlsbad, CA) according to the manufacturer's instructions.

Relative fluorescent units representing the abundance of each autoantibody in the serum of healthy and PEE pregnancies were uploaded to and analyzed with Partek's Genomics Suite (Partek Inc, St. Louis, MO). The data were quantile normalized followed by analysis of variance (ANOVA) to identify differentially expressed autoantibodies between PEE and healthy pregnancies. (Tables 4-5, Figures 5-7). Figure 5 is a graphical representation of a principal component analysis following ANOVA of 75 PEE autoantibodies, with a p value of <0.05 found to be differentially measured in serum samples from healthy pregnant women and pregnant women with PEE. From this set various combinations of autoantibodies can accurately predict PEE. If a fold change for the autoantibody is set at a threshold of >2 fold then the following 10 autoantibodieesare identified as one exemplary set to distinguish PEE: CSNK1D, GSTZ1, CDK5, PIM1, ZRANB2, PRC1, CUEDC1, SULT4A1, LOC283861, Junction Plakoglobin. Sets of 3 autoantibodies can be selected by statistical modeling to give predictive rates of PEE. CSNK1D, CUEDC1 and ZRANB2, is one exemplary set of 3 autoantibodies that gives a predictive rate of PEE with an AUC of 0.93. Another set of 3 autoantibodies, CSNK1D, SULT4A1 and Junction Plakoglobin, gives an AUC of 0.94 for PEE.

Autoantibodies with a fold change greater than 2 with a p-value with false discovery rate of less than 0.05 were considered significant and utilized to build prediction models. The Partek Genomics Suite was utilized to select a robust model for prediction of PEE among the following classification models: K-nearest neighbor, nearest centroid, discriminate analysis, support vector machine, partial least squares, diagonal discriminant analysis, random forest and logistic regression. Multiple models yielded a ROC with AUC of 0.9 or greater with as few as 3 autoantibodies. Any one or more of these models can be utilized for the diagnosis of PEE.

**Table 4: Differentially expressed autoantibodies in PEE and healthy pregnancies.**

| **No.** | **75 PEE autoantibodies (fc >,<1.5; pFDR0.05)** | | | | | **Healthy 1st Trimester** | | **Healthy 3rd Trimester** | | **Overall Healthy Pregnancy** | | **PEE** | | **PEE vs. Healthy 3rd Trimester** | | |
|---|---|---|---|---|---|---|---|---|---|---|---|---|---|---|---|---|
| | **Accession Number** | **ProtoArray Spot ID** | **Ultimate ORF ID** | **Abbreviated Name** | **Full Name** | **Mean** | **SEM** | **Mean** | **SEM** | **Mean** | **SEM** | **Mean** | **SEM** | **p-value (E is Power of 10)** | **Fold change** | **Up/Down** |
| 1 | BC010369.1 | B31R13C17(18) | IOH13610 | RNF111 | E3 ubiquitin-protein ligase Arkadia | 10.39 | 0.10 | 10.23 | 0.16 | 10.35 | 0.09 | 11.05 | 0.08 | 1.65E-04 | 1.76 | up |
| 2 | NM_ 080658.1 | B02R14C07(8) | IOH3352 | ACY3 | aspartoacylase (aminocyclase) 3 | 13.58 | 0.13 | 12.02 | 0.20 | 13.27 | 0.17 | 12.58 | 0.18 | 1.04E-01 | 1.48 | up |
| 3 | NM_ 145870.1 | B48R05C07(8) | IOH4046 | GSTZ1 | glutathione transferase zeta 1 (Maleylacetoacetate isomerase) | 13.17 | 0.14 | 12.50 | 0.20 | 13.03 | 0.13 | 14.19 | 0.22 | 8.82E-05 | 3.22 | up |
| 4 | NM_ 052822.1 | B32R13C17(18) | IOH12952 | SCAMP1 | secretory carrier membrane protein 1 | 10.16 | 0.13 | 9.68 | 0.05 | 10.06 | 0.11 | 10.91 | 0.15 | 1.95E-04 | 2.34 | up |
| 5 | XM_370653.2 | B36R02C13(14) | IOH26103 | KIAA1826 | Coiled-coil domain-containing protein K | 10.50 | 0.11 | 10.00 | 0.23 | 10.40 | 0.11 | 11.24 | 0.16 | 2.00E-04 | 2.36 | up |
| 6 | BC041563.1 | B32R16C15(16) | IOH28055 | OSBPL1A | oxysterol binding protein-like 1A | 9.72 | 0.05 | 9.97 | 0.18 | 9.77 | 0.06 | 10.46 | 0.15 | 4.85E-02 | 1.41 | up |
| 7 | NM_ 004546.1 | B47R03C03(4) | IOH4818 | NDUFB2 | NADH dehydrogenase (ubiquinone) 1 beta | 10.95 | 0.08 | 11.30 | 0.14 | 11.02 | 0.07 | 11.61 | 0.13 | 1.88E-01 | 1.24 | up |
| 8 | PV3855 | B43R15C21(22) | PV3855 | JAK3 | Tyrosine-protein kinase JAK3 | 10.07 | 0.09 | 10.41 | 0.09 | 10.14 | 0.08 | 10.73 | 0.12 | 1.70E-01 | 1.25 | up |
| 9 | BC035634.1 | B12R17C11(12) | IOH27582 | PI16 | Peptidase inhibitor 16 | 10.89 | 0.11 | 10.66 | 0.20 | 10.84 | 0.09 | 11.60 | 0.15 | 2.24E-03 | 1.92 | up |
| 10 | BC005911.1 | B44R14C17(18) | IOH7548 | SCP2 | sterol carrier protein 2 | 8.62 | 0.12 | 9.20 | 0.31 | 8.74 | 0.12 | 9.56 | 0.17 | 2.98E-01 | 1.28 | up |
| 11 | BC022459.1 | B44R12C09(10) | IOH11064 | SULT4A1 | sulfotransferase family 4A, member 1 | 12.53 | 0.14 | 12.48 | 0.13 | 12.52 | 0.11 | 13.60 | 0.23 | 8.59E-03 | 2.17 | up |
| 12 | NM_ 175899.2 | B32R06C19(20) | IOH14092 | LOC283861 | Unknown (protein for MGC: 16479) | 9.90 | 0.16 | 10.40 | 0.25 | 10.00 | 0.14 | 11.28 | 0.28 | 8.28E-02 | 1.84 | up |

| | **75 PEE autoantibodies (fc >,<1.5; pFDR0.05)** | | | | | **Healthy 1st Trimester** | | **Healthy 3rd Trimester** | | **Overall Healthy Pregnancy** | | **PEE** | | **PEE vs. Healthy 3rd Trimester** | | |
|---|---|---|---|---|---|---|---|---|---|---|---|---|---|---|---|---|
| | **Accession Number** | **ProtoArray Spot ID** | **Ultimate ORF ID** | **Abbreviated Name** | **Full Name** | **Mean** | **SEM** | **Mean** | **SEM** | **Mean** | **SEM** | **Mean** | **SEM** | **p₋value (E is Power of 10)** | **Fold change** | **Up/Down** |
| 13 | BC053351.1 | B24R17C07(8) | IOH28952 | DLX1 | distal-less homeobox 1 | 9.44 | 0.09 | 10.22 | 0.20 | 9.60 | 0.10 | 10.34 | 0.20 | 7.36E-01 | 1.08 | up |
| 14 | NM_ 002279.3 | B01R05C13(14) | IOH13096 | KRT33B | keratin 33B | 11.45 | 0.15 | 10.68 | 0.10 | 11.29 | 0.14 | 10.81 | 0.08 | 5.97E-01 | 1.10 | up |
| 15 | PV3758 | B28R15C15(16) | PV3758 | PRKD2 | protein kinase D2, transcript v | 10.57 | 0.11 | 10.63 | 0.30 | 10.58 | 0.10 | 11.55 | 0.22 | 2.10E-02 | 1.90 | up |
| 16 | BC010959.1 | B47R13C09(10) | IOH13742 | BNIP1 | BCL2/adenovirus E1B 19kDa interacting protein | 10.32 | 0.07 | 10.21 | 0.14 | 10.30 | 0.06 | 10.91 | 0.15 | 7.39E-03 | 1.62 | up |
| 17 | Hs∼IVGN:PM 2136∼Ext:U1sn RNP68 | B18R15C13(14) | NA | U1snRNP68 | Small nuclear ribonucleoprotein complex 68 | 9.14 | 0.06 | 8.35 | 0.07 | 8.98 | 0.08 | 8.39 | 0.19 | 9.10E-01 | 1.02 | up |
| 18 | BC015667.2 | B19R14C19(20) | IOH14654 | KLHL29 | kelch-like 29 (Drosophila) | 12.71 | 0.13 | 12.29 | 0.22 | 12.63 | 0.12 | 13.40 | 0.17 | 1.78E-03 | 2.16 | up |
| 19 | NM_ 004577.3 | B32R05C21(22) | IOH39828 | PSPH | phosphoserine phosphatase | 13.18 | 0.14 | 13.02 | 0.27 | 13.15 | 0.12 | 13.91 | 0.15 | 7.82E-03 | 1.85 | up |
| 20 | BC025404.1 | B08R06C21(22) | IOH11756 | MEIS3 | Meis homeobox 3, transcript var | 9.94 | 0.16 | 9.68 | 0.11 | 9.89 | 0.13 | 10.89 | 0.23 | 6.30E-03 | 2.30 | up |
| 21 | NM_ 023076.2 | B35R03C17(18) | IOH2885 | C16orf28 | RING finger protein unkempt-like | 11.02 | 0.12 | 10.76 | 0.05 | 10.97 | 0.10 | 11.58 | 0.13 | 3.36E-03 | 1.76 | up |
| 22 | NM_ 002230.1 | B08R19C19(20) | IOH54131 | JUP | Junction plakoglobin | 10.23 | 0.19 | 10.44 | 0.13 | 10.27 | 0.15 | 11.37 | 0.24 | 5.39E-02 | 1.90 | up |
| 23 | BC022004.2 | B31R04C15(16) | IOH11222 | CTNNA3 | catenin (cadherin-associated protein), alpha 3 | 10.52 | 0.11 | 10.52 | 0.13 | 10.52 | 0.09 | 11.21 | 0.16 | 2.29E-02 | 1.62 | up |
| 24 | NM_ 017949.1 | B28R09C13(14) | IOH29099 | CUEDC1 | CUE domain containing 1 | 12.64 | 0.15 | 13.68 | 0.51 | 12.85 | 0.18 | 13.74 | 0.24 | 8.90E-01 | 1.05 | up |
| 25 | BC007530.1 | B44R16C17(18) | IOH6875 | EMILIN1 | elastin microfibril interfacer 1 | 10.24 | 0.12 | 10.51 | 0.19 | 10.30 | 0.10 | 11.00 | 0.16 | 1.26E-01 | 1.40 | up |
| 26 | BC010889.1 | B31R13C09(10) | IOH12754 | CCDC53 | coiled-coil domain containing 53 | 9.57 | 0.10 | 10.13 | 0.36 | 9.69 | 0.11 | 10.32 | 0.16 | 5.44E-01 | 1.14 | up |
| 27 | NM_ 024818.1 | B16R06C17(18) | IOH9860 | UBE1DC1 | ubiquitin-acti vating enzyme E1-domain containing 1 | 9.85 | 0.13 | 10.32 | 0.27 | 9.94 | 0.12 | 10.73 | 0.20 | 2.68E-01 | 1.33 | up |
| 28 | PV4393 | B08R15C19(20) | PV4393 | JAK2 | Janus kinase 2 (a protein tyrosine kinase) | 10.05 | 0.16 | 10.55 | 0.32 | 10.15 | 0.14 | 10.91 | 0.16 | 3.27E-01 | 1.28 | up |
| 29 | NM_ 001250.2 | B32R14C21(22) | IOH10427 | CD40 | CD40 molecule, TNF receptor superfamily | 10.65 | 0.13 | 10.48 | 0.18 | 10.62 | 0.11 | 11.30 | 0.14 | 1.01E-02 | 1.76 | up |
| 30 | NM_ 006136.1 | B24R04C07(8) | IOH7253 | CAPZA2 | capping protein (actin filament) muscle | 11.00 | 0.11 | 11.35 | 0.23 | 11.07 | 0.10 | 11.69 | 0.15 | 2.44E-01 | 1.27 | up |
| 31 | BC058899.1 | B20R07C01(2) | IOH29087 | DSN1 | DSN1, MIND kinetochore complex component | 11.69 | 0.13 | 11.41 | 0.19 | 11.64 | 0.11 | 12.43 | 0.20 | 7.68E-03 | 2.02 | up |
| 32 | NM_ 194310.1 | B48R07C09(10) | IOH35456 | LOC284837 | hypothetical protein LOC284837 | 11.98 | 0.10 | 11.77 | 0.12 | 11.93 | 0.09 | 12.57 | 0.16 | 9.20E-03 | 1.74 | up |
| 33 | BC005951.1 | B48R04C19(20) | IOH7490 | IGHA1 | immunoglobulin heavy constant alpha 1 | 10.25 | 0.15 | 10.10 | 0.09 | 10.22 | 0.12 | 10.89 | 0.14 | 1.38E-02 | 1.74 | up |
| 34 | BC034052.1 | B36R11C21(22) | IOH22224 | DACT3 | Dapper homolog 3 | 11.77 | 0.17 | 11.86 | 0.23 | 11.79 | 0.14 | 12.58 | 0.17 | 5.75E-02 | 1.65 | up |
| 35 | BC013116.1 | B37R07C17(18) | IOH28618 | CLIP3 | CAP-Gly domain-containing linker protein | 11.74 | 0.18 | 12.79 | 0.13 | 11.95 | 0.17 | 11.06 | 0.09 | 1.34E-06 | -3.32 | down |
| 36 | PV3269 | B25R16C01(2) | PV3269 | TEC | tec protein tyrosine kinase | 9.66 | 0.19 | 10.74 | 0.40 | 9.87 | 0.19 | 8.87 | 0.09 | 2.42E-06 | -3.67 | down |
| 37 | NM_ 148976.1 | B17R21C15(16) | IOH41126 | PSMA1 | Proteasome subunit alpha type-1 | 10.53 | 0.16 | 11.56 | 0.41 | 10.74 | 0.17 | 9.74 | 0.13 | 3.67E-06 | -3.52 | down |
| 38 | NM_ 005678.3 | B05R09C11(12) | IOH45840 | SNURF | SNRPN upstream reading frame | 12.61 | 0.15 | 13.46 | 0.39 | 12.78 | 0.16 | 11.86 | 0.14 | 2.81E-05 | -3.04 | down |
| 39 | PV3248 | B05R15C17(18) | PV3248 | CSNK2A1 | casein kinase 2, alpha 1 polypeptide | 9.33 | 0.11 | 9.95 | 0.48 | 9.45 | 0.13 | 8.68 | 0.12 | 9.29E-05 | -2.41 | down |
| 40 | PV3665 | B05R16C01(2) | PV3665 | CSNK1D | casein kinase 1, delta | 10.76 | 0.27 | 11.40 | 0.24 | 10.89 | 0.22 | 9.58 | 0.11 | 1.71E-04 | -3.53 | down |
| 41 | NM_ 005163.1 | B25R16C05(6) | PV3685 | AKT1 | RAC-beta serine/threonine-protein kinase | 8.59 | 0.18 | 9.11 | 0.18 | 8.69 | 0.15 | 7.83 | 0.10 | 2.65E-04 | -2.43 | down |
| 42 | NM_ 021123.1 | B05R13C01(2) | IOH27363 | GAGE7B | G antigen 7 | 10.12 | 0.18 | 10.60 | 0.46 | 10.21 | 0.17 | 9.32 | 0.10 | 7.00E-04 | -2.43 | down |
| 43 | BC020814.1 | B06R12C01(2) | IOH12692 | SUGT1L1 | SGT1, suppressor of G2 allele of SKP1 1 | 11.57 | 0.14 | 11.94 | 0.36 | 11.65 | 0.13 | 10.92 | 0.10 | 8.78E-04 | -2.03 | down |
| 44 | NM_ 003041.1 | B05R21C13(14) | IOH38149 | SLC5A2 | Sodium/glucose cotransporter 2 | 9.55 | 0.11 | 9.79 | 0.14 | 9.60 | 0.09 | 8.99 | 0.10 | 1.03E-03 | -1.74 | down |
| 45 | NM_ 002128.2 | B10R15C03(4) | IOH2937 | HMGB1 | high-mobility group box 1 | 11.63 | 0.11 | 11.94 | 0.20 | 11.69 | 0.10 | 10.98 | 0.15 | 1.61E-03 | -1.95 | down |
| 46 | NM_ 021639.2 | B29R14C03(4) | IOH10045 | GPBP1L1 | GC-rich promoter binding protein 1-like | 12.44 | 0.14 | 12.79 | 0.63 | 12.51 | 0.16 | 11.58 | 0.13 | 1.79E-03 | -2.31 | down |
| 47 | BC008145.1 | B13R14C21(22) | IOH3304 | APEX1 | APEX nuclease (multifunctional DNA repair enzyme) | 10.99 | 0.11 | 11.00 | 0.05 | 10.99 | 0.09 | 10.37 | 0.09 | 5.93E-03 | -1.55 | down |
| 48 | NM_ 001031.4 | B10R18C03(4) | IOH58930 | RPS28 | 40S ribosomal protein S28 | 12.63 | 0.12 | 12.73 | 0.17 | 12.65 | 0.10 | 11.90 | 0.16 | 9.25E-03 | -1.78 | down |
| 49 | NM_ 001014.2 | B01R16C19(20) | IOH4063 | RPS10 | ribosomal protein S10 | 11.94 | 0.11 | 12.12 | 0.10 | 11.97 | 0.09 | 11.39 | 0.14 | 9.59E-03 | -1.67 | down |
| 50 | BC006550.1 | B13R16C13(14) | IOH6089 | RBMX | RNA binding motif protein, X-linked | 10.33 | 0.15 | 10.44 | 0.14 | 10.35 | 0.12 | 9.77 | 0.08 | 1.16E-02 | -1.60 | down |
| 51 | NM_ 052969.1 | B10R08C07(8) | IOH12514 | RPL39L | ribosomal protein L39-like | 13.06 | 0.10 | 13.22 | 0.13 | 13.09 | 0.09 | 12.48 | 0.16 | 1.17E-02 | -1.68 | down |
| 52 | BC057772.1 | B05R19C11(12) | IOH29154 | C18orf22 | chromosome 18 open reading frame 22 | 10.22 | 0.15 | 10.30 | 0.09 | 10.24 | 0.12 | 9.58 | 0.10 | 1.24E-02 | -1.64 | down |
| 53 | NM_ 138551.1 | B05R05C05(6) | IOH13700 | TSLP | thymic stromal lymphopoietin | 11.74 | 0.10 | 11.88 | 0.02 | 11.77 | 0.08 | 11.18 | 0.15 | 1.27E-02 | -1.63 | down |
| 54 | NM_ 007227.3 | B06R19C05(6) | IOH40151 | GPR45 | G protein-coupled receptor 45 | 8.97 | 0.16 | 8.95 | 0.10 | 8.97 | 0.13 | 8.23 | 0.09 | 1.37E-02 | -1.65 | down |
| 55 | PV3503 | B01R16C01(2) | PV3503 | PIM1 | pim-1 oncogene | 8.89 | 0.20 | 8.58 | 0.21 | 8.83 | 0.16 | 7.46 | 0.21 | 1.59E-02 | -2.17 | down |
| 56 | BC030290.1 | B13R14C11(12) | IOH21571 | RBMS3 | RNA-binding motif, single-stranded-interacting protein 3 | 11.12 | 0.15 | 11.13 | 0.12 | 11.12 | 0.13 | 10.46 | 0.11 | 2.64E-02 | -1.59 | down |
| 57 | BC018749.1 | B02R20C09(10) | IOH14788 | IGLV2-14 | immunoglobulin lambda variable 2-14 | 10.63 | 0.12 | 10.65 | 0.06 | 10.63 | 0.09 | 10.05 | 0.13 | 2.65E-02 | -1.52 | down |
| 58 | PHC1244 | B26R16C05(6) | PHC1244 | CCL19 | chemokine (C-C motif) ligand 19 | 12.16 | 0.17 | 12.12 | 0.19 | 12.15 | 0.14 | 11.36 | 0.14 | 3.26E-02 | -1.69 | down |
| 59 | PV3145 | B09R15C15(16) | PV3145 | FGFR3 | fibroblast growth factor receptor 3 | 8.87 | 0.10 | 8.65 | 0.21 | 8.83 | 0.09 | 8.03 | 0.15 | 3.66E-02 | -1.53 | down |
| 60 | NM_ 024668.1 | B15R12C09(10) | IOH5012 | ANKHD1 | ankyrin repeat and KH domain containing | 10.69 | 0.12 | 10.38 | 0.26 | 10.62 | 0.11 | 9.81 | 0.13 | 4.60E-02 | -1.48 | down |
| 61 | NM_ 007099.1 | B48R03C09(10) | IOH6098 | ACP1 | acid phosphatase 1, soluble | 12.08 | 0.13 | 13.41 | 0.33 | 12.35 | 0.16 | 12.88 | 0.16 | 1.12E-01 | -1.45 | down |
| 62 | NM_00101297 8.1 | B19R21C19(20) | IOH42168 | BEX5 | Protein BEX5 | 9.44 | 0.18 | 10.71 | 0.29 | 9.69 | 0.19 | 10.24 | 0.12 | 1.77E-01 | -1.39 | down |
| 63 | NM_ 006708.1 | B08R21C07(8) | IOH10090 | GLO1 | Lactoylglutathione lyase | 11.00 | 0.14 | 12.00 | 0.13 | 11.20 | 0.14 | 11.66 | 0.11 | 2.14E-01 | -1.27 | down |
| 64 | PV4786 | B11R16C03(4) | PV4786 | PIK3CG | class IB phosphatidylinositol 3-kinase | 8.83 | 0.18 | 8.27 | 0.13 | 8.71 | 0.15 | 7.89 | 0.11 | 2.47E-01 | -1.30 | down |
| 65 | XM_ 498434.1 | B20R19C19(20) | IOH57016 | MGC16075 | hypothetical protein MGC16075, mRNA | 10.67 | 0.16 | 11.89 | 0.35 | 10.92 | 0.17 | 11.52 | 0.16 | 2.97E-01 | -1.29 | down |
| 66 | NM_ 003981.2 | B45R09C17(18) | IOH5138 | PRC1 | protein regulator of cytokinesis 1 | 15.21 | 0.22 | 14.44 | 0.15 | 15.06 | 0.19 | 14.07 | 0.16 | 3.60E-01 | -1.30 | down |
| 67 | NM_ 203350.1 | B17R05C21(22) | IOH36832 | ZRANB2 | zinc finger, RAN-binding domain containing 2 | 12.45 | 0.19 | 11.78 | 0.27 | 12.31 | 0.17 | 11.45 | 0.15 | 3.90E-01 | -1.26 | down |
| 68 | BC039814.1 | B09R18C09(10) | IOH26283 | ZRANB2 | zinc finger, RAN-binding domain containing 2 | 11.93 | 0.21 | 11.08 | 0.35 | 11.76 | 0.19 | 10.73 | 0.23 | 4.69E-01 | -1.28 | down |
| 69 | NM_ 005347.2 | B18R14C07(8) | IOH14762 | HSPA5 | heat shock 70kDa protein 5 (glucose-reg | 10.81 | 0.13 | 10.33 | 0.07 | 10.71 | 0.11 | 10.19 | 0.08 | 5.34E-01 | -1.10 | down |
| 70 | PV4676 | B39R16C05(6) | PV4676 | CDK5 | cyclin-dependent kinase 5 | 8.66 | 0.20 | 10.85 | 0.42 | 9.10 | 0.25 | 10.53 | 0.35 | 6.08E-01 | -1.25 | down |
| 71 | BC053898.1 | B01R19C17(18) | IOH29029 | HOXC8 | homeobox C8 | 10.35 | 0.08 | 9.82 | 0.12 | 10.24 | 0.08 | 9.71 | 0.12 | 6.28E-01 | -1.08 | down |
| 72 | NM_ 006573.2 | B05R03C13(14) | IOH12947 | TNFSF13B | tumor necrosis factor (ligand) superfam | 13.67 | 0.11 | 13.14 | 0.15 | 13.56 | 0.10 | 13.04 | 0.14 | 7.00E-01 | -1.07 | down |
| 73 | NM_ 003168.1 | B23R03C05(6) | IOH5411 | SUPT4H1 | suppressor of Ty 4 homolog 1 | 14.05 | 0.18 | 13.26 | 0.25 | 13.89 | 0.16 | 13.21 | 0.14 | 9.00E-01 | -1.03 | down |
| 74 | NM_ 014321.2 | B22R05C13(14) | IOH39827 | ORC6L | origin recognition complex, subunit 6 1 | 12.31 | 0.19 | 11.48 | 0.08 | 12.15 | 0.17 | 11.45 | 0.12 | 9.21E-01 | -1.02 | down |
| 75 | BC051695.1 | B17R09C11(12) | IOH26532 | FKSG44 | FERM domain containing 8 | 13.67 | 0.11 | 13.03 | 0.27 | 13.54 | 0.11 | 13.01 | 0.14 | 9.43E-01 | -1.01 | down |

**Table 5: One set of 10 autoantibodies differentially expressed in PEE and healthy pregnancies**

| **10 Differentially Expressed Autoantibodies (fc >,<1.5; pFDR0.05)** | | | | | **Healthy 1st Trimester** | | **Healthy 3rd Trimester** | | **Overall Healthy Pregnancy** | | **PEE** | | **PEE vs. Healthy 3rd Trimester** | | |
|---|---|---|---|---|---|---|---|---|---|---|---|---|---|---|---|
| **Accession Number** | **ProtoArray Spot ID** | **Ultimate ORF ID** | **Abbreviated Name** | **Full Name** | **Mean (log2)** | **SEM** | **Mean (log2)** | **SEM** | **Mean (log2)** | **SEM** | **Mean (log2)** | **SEM** | **p-value (E is Power of 10)** | **Fold change (log2)** | **Up/Down** |
| NM_145870.1 | B48R05C07(8) | IOH4046 | GSTZ1 | glutathione transferase zeta 1 (Maleylacetoacetate isomerase) | 13.17 | 0.14 | 12.50 | 0.20 | 13.03 | 0.13 | 14.19 | 0.22 | 8.82E-05 | 3.22 | up |
| BC022459.1 | B44R12C09(10) | IOH11064 | SULT4A1 | sulfotransferase family 4A, member 1 | 12.53 | 0.14 | 12.48 | 0.13 | 12.52 | 0.11 | 13.60 | 0.23 | 8.59E-03 | 2.17 | up |
| NM_002230.1 | B08R19C19(20) | IOH54131 | JUP | Junction plakoglobin | 10.23 | 0.19 | 10.44 | 0.13 | 10.27 | 0.15 | 11.37 | 0.24 | 5.39E-02 | 1.90 | up |
| NM_017949.1 | B28R09C13(14) | IOH29099 | CUEDC1 | CUE domain containing 1 | 12.64 | 0.15 | 13.68 | 0.51 | 12.85 | 0.18 | 13.74 | 0.24 | 8.90E-01 | 1.05 | up |
| NM_194310.1 | B48R07C09(10) | IOH35456 | LOC284837 | hypothetical protein LOC284837 | 11.98 | 0.10 | 11.77 | 0.12 | 11.93 | 0.09 | 12.57 | 0.16 | 9.20E-03 | 1.74 | up |
| PV3665 | B05R16C01(2) | PV3665 | CSNK1D | casein kinase 1, delta | 10.76 | 0.27 | 11.40 | 0.24 | 10.89 | 0.22 | 9.58 | 0.11 | 1.71E-04 | -3.53 | down |
| PV3503 | B01R16C01(2) | PV3503 | PIM1 | pim-1 oncogene | 8.89 | 0.20 | 8.58 | 0.21 | 8.83 | 0.16 | 7.46 | 0.21 | 1.59E-02 | -2.17 | down |
| NM_003981.2 | B45R09C17(18) | IOH5138 | PRC1 | protein regulator of cytokinesis 1 | 15.21 | 0.22 | 14.44 | 0.15 | 15.06 | 0.19 | 14.07 | 0.16 | 3.60E-01 | -1.30 | down |
| BC039814.1 | B09R18C09(10) | IOH26283 | ZRANB2 | zinc finger, RAN-binding domain containing 2 | 11.93 | 0.21 | 11.08 | 0.35 | 11.76 | 0.19 | 10.73 | 0.23 | 4.69E-01 | -1.28 | down |
| PV4676 | B39R16C05(6) | PV4676 | CDK5 | cyclin-dependent kinase 5 | 8.66 | 0.20 | 10.85 | 0.42 | 9.10 | 0.25 | 10.53 | 0.35 | 6.08E-01 | -1.25 | down |

### Example 3: Prediction of PEE in a sample from a pregnant woman

A 50 milliliter blood sample is taken from a pregnant woman who presents with high blood pressure at week 13 or later of her pregnancy, and is therefore suspected of either having or developing PEE. Blood is coagulated to yield serum. The serum sample is depleted of 20 of the most abundant serum proteins with ProteoPrep20 Plasma Immunodepletion Kit (Sigma-Aldrich, Cat.no PROT20).

Serum proteins are then identified and quantitate using an enzyme-linked immunosorbant assay (ELISA), using standard protocols. In one embodiment antibodies specific for the following proteins can be used in the ELISA assay: APCS, THBS1, PRG2, ITIH4, GSN, HPX, F5, and TF.

In another alternative assay, the serum proteins are identified and quantified using a spectral imager.

In yet another assay, the serum proteins are identified and quantitated using a MagArray protein chip. For the MagArray protein chip, the serum is applied onto the chip where probes on the surface specifically bind to serum proteins in the sample. Second, nanotag-labeled antibodies specific to proteins of interest bind to the serum proteins bound to the probes, forming sandwich-like structures. Third, an external magnetic field is applied to the chip and the stray magnetic field produced by the nanotags is measured electrically to determine the presence and amount of each of the proteins of interest. In one embodiment antibodies specific for the following target proteins can be used in the MagArray assay: APCS, THBS1, PRG2, ITIH4, GSN, HPX, F5, and TF. In one assay format, 3 targets from the preceding list are selected for detection and quantification and used in the assay to form the sandwich like structures. Binding data for the 3 proteins are quantified. Concentrations of the proteins are determined, and compared to reference sample concentration values, taken from the serum of a healthy pregnant woman of approximately the same gestational age.

In still another assay, carboxyl bead sets can be used to measure proteins, peptides, and autoantibodies of interest. In this assay, any protein or peptide can be covalently attached to a highly stable microbead surface followed by fluorescent labeling and fluorescence intensity measurement. The VeraCode Technology by Illumina (Illumina Inc., Hayward, CA) allows one to perform up to 48 immunoassays in varying combinations in a single reaction in a standard 96-well microplate.

In yet another alternative, proteins, peptides and autoantibodies can be measured by a electrochemiluminesence ELISA. The sensitive multiplexed electrochemiluminesence ELISA platform by Meso Scale Discovery (MSD, Gaithersburg, MD) is a high throughput multiplexed ELISA for custom designed use with the capability to simultaneously measure up to 10 analytes in the same well.

Any change in the concentration (upregulation or downregulation) of the 3 proteins in the serum sample taken from a woman suspected of having PEE, as compared to the level of those proteins in the serum sample from a healthy pregnant woman, is indicative of a diagnosis of PEE. This result is then transmitted back to the pregnant woman's physician and appropriate treatment measures can be taken to protect the pregnant mother and the growing fetus.

The present application provides the following particular embodiments:
1. A method for detecting the risk of developing preeclampsia in a pregnant woman, the method comprising
   a. contacting a biological sample from the woman with a binding agent; and
   b. detecting the binding of the binding agent to at least three PEE proteins present in the sample, wherein the at least three PEE proteins are selected from the proteins listed in Table 1, the proteins listed in Table 2, or the combinations of proteins in Table 3;
   wherein the binding agent binds the PEE proteins with a K_{d} of 10⁻¹² M to 10-⁵ M, and wherein the binding of the binding agent to the PEE proteins in the sample is increased as compared to the binding of the binding agent to the PEE proteins in a biological sample from a healthy pregnant woman in the same trimester, whereby the increase in binding indicates the risk of developing preeclampsia to at least a 80% degree of accuracy.
2. The method of item 1 wherein the binding agent further binds a PEE protein selected from Table 1 or Table 2.
3. The method of item 1 wherein the binding agent binds a PEE protein selected from Table 1.
4. The method of item 1 wherein the binding agent binds a PEE protein selected from Table 2.
5. The method of item 2 wherein the binding agent binds no more than 48 PEE proteins.
6. The method of item 1 wherein the woman is in the third trimester of her pregnancy.
7. The method of item 1 wherein the woman is in the second trimester of her pregnancy.
8. The method of item 1 wherein the woman is in the first trimester of her pregnancy.
9. The method of item 1 wherein the biological sample is a non-fetal maternal sample.
10. The method of item 1 wherein the biological sample is urine, blood, or placental tissue.
11. The method of item 1 wherein the increase in binding indicates the risk of developing preeclampsia to at least a 90% degree of accuracy.
12. The method of item 1 wherein the increase in binding indicates the risk of developing preeclampsia to at least a 95% degree of accuracy.
13. The method of item 1 wherein the binding agent comprises a mixture of individual antibodies to the PEE proteins GSN, THBS1 and PRG2.
14. The method of item 1 wherein the binding agent further binds a PEE autoantibody present in the sample.
15. The method of item 14 wherein the binding agent further binds a PEE autoantibody selected from Table 4 or Table 5.
16. The method of item 14 wherein the binding agent binds PEE autoantibodies to CSNK1D, CUEDC1 and ZRANB2.
17. The method of item 14 wherein the binding agent binds PEE autoantibodies to CSNK1D, SULT4A1 and Junction Plakoglobin.
18. The method of item 1 wherein the binding agent comprises an antibody.
19. The method of item 1 wherein the binding agent comprises a nucleoprotein.
20. The method of item 1 wherein the binding agent comprises a peptide.
21. The method of item 1 wherein the binding agent comprises a fluorescent label.
22. The method of item 1 wherein the binding agent comprises a labeled antibody.
23. The method of item 1 wherein the binding is carried out by an immunoassay.
24. The method of item 23 wherein the immunoassay is an enzyme-linked immunosorbent assay.
25. The method of item 23 wherein the immunoassay comprises beads.
26. The method of item 23 wherein the immunoassay comprises magnetic particles.
27. The method of item 23 wherein the immunoassay does not comprise magnetic particles.
28. The method of item 1 wherein the binding agent is a nucleoprotein comprising protein binding sites.
29. The method of item 1 wherein the method is further used for predicting the onset of preeclampsia, monitoring the progression of preeclampsia , monitoring the regression of preeclampsia, assessing efficacy of treatment for preeclampsia, identifying a sub-population of patients who should be treated for preeclampsia or identifying a sub-population of patients who should be monitored for preeclampsia symptoms.
30. A method for detecting the risk of developing preeclampsia in a pregnant woman, the method comprising
   a. contacting a biological sample from the woman with a binding agent; and
   b. detecting the binding of the binding agent to a PEE autoantibody present in the sample;
   wherein the binding agent binds the PEE autoantibody with a K_{d} of 10⁻¹² M to 10⁻⁵ M, and wherein the binding of the binding agent to the PEE autoantibody in the sample is changed as compared to the binding of the binding agent to the PEE autoantibody in a biological sample from a healthy pregnant woman in the same trimester, whereby the change in binding indicates the risk of developing preeclampsia to at least a 80% degree of accuracy.
31. The method of item 30 wherein the PEE autoantibody is selected from Table 4 or Table 5.
32. The method of item 30 wherein the binding agent comprises a protein.
33. The method of item 32 wherein the binding agent comprises a protein comprising a label.
34. The method of item 33 wherein the label is a fluorescent label.
35. The method of item 30 wherein the binding agent comprises a protein array.
36. The method of item 30 wherein the binding of the binding agent to the PEE autoantibody in the sample is increased as compared to the binding of the binding agent to the autoantibody in a biological sample from a healthy pregnant woman.
37. The method of item 30 wherein the binding of the binding agent to the PEE autoantibody in the sample is decreased as compared to the binding of the binding agent to the autoantibody in a biological sample from a healthy pregnant woman.
38. The method of item 30 wherein the binding agent binds PEE autoantibodies to CSNK1D, CUEDC1 and ZRANB2.
39. The method of item 30 wherein the binding agent binds PEE autoantibodies to CSNK1D, SULT4A1 and Junction Plakoglobin.
40. The method of item 30 wherein the binding agent further binds a PEE protein selected from Table 1 or Table 2.
41. The method of item 30 wherein the binding agent further binds a PEE protein selected from GSN, THBS1 or PRG2.
42. The method of item 40 wherein the binding agent binds no more than 48 PEE proteins.
43. The method of item 30 wherein the woman is in the third trimester of her pregnancy.
44. The method of item 30 wherein the woman is in the second trimester of her pregnancy.
45. The method of item 30 wherein the woman is in the first trimester of her pregnancy.
46. The method of item 30 wherein the biological sample is a non-fetal maternal sample.
47. The method of item 30 wherein the biological sample is urine, blood, or placental tissue.
48. The method of item 30 wherein the increase in binding indicates the risk of developing preeclampsia to at least a 90% degree of accuracy.
49. The method of item 30 wherein the increase in binding indicates the risk of developing preeclampsia to at least a 95% degree of accuracy.
50. The method of item 30 wherein the binding is carried out by an immunoassay.
51. The method of item 50 wherein the immunoassay is an enzyme-linked immunosorbent assay.
52. The method of item 50 wherein the immunoassay comprises beads.
53. The method of item 50 wherein the immunoassay comprises magnetic particles.
54. The method of item 50 wherein the immunoassay does not comprise magnetic particles.
55. The method of item 30 wherein the method is further used for predicting the onset of preeclampsia, monitoring the progression of preeclampsia , monitoring the regression of preeclampsia, assessing efficacy of treatment for preeclampsia, identifying a sub-population of patients who should be treated for preeclampsia or identifying a sub-population of patients who should be monitored for preeclampsia symptoms.
56. A method for detecting the risk of developing preeclampsia in a pregnant woman, the method comprising
   a. contacting a biological sample from the woman with a binding agent; and
   b. detecting the binding of the binding agent to at least one PEE protein and at least one PEE autoantibody present in the sample;
   wherein the binding agent binds the at least one PEE protein with a K_{d} of 10⁻¹² M to 10⁻⁵ M, wherein the binding agent binds the at least one PEE autoantibody with a K_{d} of 10⁻¹² M to 10⁻⁵ M, and wherein the binding of the binding agent to the at least one PEE protein and the at least one PEE autoantibody in the sample is changed as compared to the binding of the binding agent to the at least one PEE protein and at least one PEE autoantibody in a biological sample from a healthy pregnant woman in the same trimester, whereby the change in binding indicates the risk of developing preeclampsia to at least a 80% degree of accuracy.
57. The method of item 56 wherein the autoantibody is selected from Table 4 or Table 5.
58. The method of item 56 wherein the binding of the binding agent to the PEE autoantibody in the sample is increased as compared to the binding of the binding agent to the PEE autoantibody in a biological sample from a healthy pregnant woman.
59. The method of item 56 wherein the binding of the binding agent to the PEE autoantibody in the sample is decreased as compared to the binding of the binding agent to the PEE autoantibody in a biological sample from a healthy pregnant woman.
60. The method of item 56 wherein the protein is selected from Table 1 or Table 2.
61. The method of item 56 wherein the binding agent further binds a PEE protein selected from GSN, THBS1 or PRG2.
62. The method of item 56 wherein the binding agent binds more than one PEE protein.
63. The method of item 62 wherein the binding agent further binds the PEE proteins GSN, THBS1 and PRG2.
64. The method of item 62 wherein the binding agent binds no more than 48 PEE proteins.
65. The method of item 56 wherein the binding agent binds more than one PEE autoantibody.
66. The method of item 65 wherein the binding agent binds PEE autoantibodies to CSNK1D, CUEDC1 and ZRANB2.
67. The method of item 65 wherein the binding agent binds PEE autoantibodies to CSNK1D, SULT4A1 and Junction Plakoglobin.
68. The method of item 56 wherein the woman is in the third trimester of her pregnancy.
69. The method of item 56 wherein the woman is in the second trimester of her pregnancy.
70. The method of item 56 wherein the woman is in the first trimester of her pregnancy.
71. The method of item 56 wherein the biological sample is a non-fetal maternal sample.
72. The method of item 56 wherein the biological sample is urine, blood, or placental tissue.
73. The method of item 56 wherein the increase in binding indicates the risk of developing preeclampsia to at least a 90% degree of accuracy.
74. The method of item 56 wherein the increase in binding indicates the risk of developing preeclampsia to at least a 95% degree of accuracy.
75. The method of item 56 wherein the binding agent comprises a labeled antibody and a labeled protein.
76. The method of item 56 wherein the label is a fluorescent label.
77. The method of item 56 wherein the binding is carried out by an immunoassay.
78. The method of item 56 wherein the immunoassay is an enzyme-linked immunosorbent assay.
79. The method of item 77 wherein the immunoassay comprises beads.
80. The method of item 77 wherein the immunoassay comprises magnetic particles.
81. The method of item 77 wherein the immunoassay does not comprise magnetic particles.
82. The method of item 56 wherein the method is further used for predicting the onset of preeclampsia, monitoring the progression of preeclampsia, monitoring the regression of preeclampsia, or assessing efficacy of treatment for preeclampsia.
83. A composition comprising one or more solid surfaces comprising binding agents for GSN, THBS1 and PRG2.
84. A composition comprising one or more solid surfaces comprising binding agents for CSNK1D, CUEDC1 and ZRANB2 PEE autoantibodies.
85. A composition comprising one or more solid surfaces comprising binding agents for CSNK1D, SULT4A1 and Junction Plakoglobin PEE autoantibodies.
86. A diagnostic assay kit comprising:
   a. reagents for detecting GSN, THBS1 and PRG2 in a biological sample from a pregnant woman;
   b. a composition comprising one or more solid surfaces that contain one or more binding agents for GSN, THBS1 and PRG2; and
   c. instructions for use of the assay.
87. A diagnostic assay kit comprising:
   a. reagents for detecting a PEE autoantibody in a biological sample from a pregnant woman;
   b. a composition comprising one or more solid surfaces that contain one or more binding agents for the PEE autoantibody; and
   c. instructions for use of the assay.
88. The kit of item 87 wherein the composition comprises a solid surface capable of binding the CSNK1D, CUEDC1 and ZRANB2 PEE autoantibodies.
89. The kit of item 87 wherein the composition comprises a solid surface capable of binding the CSNK1D, SULT4A1 and Junction Plakoglobin PEE autoantibodies.

## Claims

1. A method for detecting the risk of developing preeclampsia in a pregnant woman, the method comprising
a. contacting a biological sample from the woman with a binding agent; and
b. detecting the binding of the binding agent to a PEE autoantibody present in the sample;
wherein the binding agent binds the PEE autoantibody with a K_{d} of 10⁻¹² M to 10⁻⁵M, and wherein the binding of the binding agent to the PEE autoantibody in the sample is changed as compared to the binding of the binding agent to the PEE autoantibody in a biological sample from a healthy pregnant woman in the same trimester, whereby the change in binding indicates the risk of developing preeclampsia to at least a 80% degree of accuracy.

2. The method of claim 1, wherein the PEE autoantibody is selected from Table 4 or Table 5.

3. The method of claim 1, wherein the binding agent comprises
A) a protein, preferably a protein comprising a label, wherein the label is preferably a fluorescent label; or
B) a protein array.

4. The method of claim 1, wherein the binding of the binding agent to the PEE autoantibody in the sample is increased as compared to the binding of the binding agent to the autoantibody in a biological sample from a healthy pregnant woman,
or wherein the binding of the binding agent to the
PEE autoantibody in the sample is decreased as compared to the binding of the binding agent to the autoantibody in a biological sample from a healthy pregnant woman.

5. The method of claim 1, wherein the binding agent binds PEE autoantibodies to CSNK1D, CUEDC1 and ZRANB2 or PEE autoantibodies to CSNK1D, SULT4A1 and Junction Plakoglobin.

6. The method of claim 1, wherein
A) the binding agent further binds a PEE protein selected from Table 1 or Table 2, wherein, preferably, the binding agent binds no more than 48 PEE proteins, or
B) the binding agent further binds a PEE protein selected from GSN, THBS1 or PRG2.

7. The method of claim 1, wherein the woman is in the third, second or first trimester of her pregnancy.

8. The method of claim 1, wherein the biological sample is a non-fetal maternal sample or wherein the biological sample is urine, blood, or placental tissue.

9. The method of claim 1, wherein the increase in binding indicates the risk of developing preeclampsia to at least a 90%, preferably at least a 95% degree of accuracy.

10. The method of claim 1, wherein the binding is carried out by an immunoassay.

11. The method of claim 10, wherein
- the immunoassay is an enzyme-linked immunosorbent assay;
- the immunoassay comprises beads;
- the immunoassay comprises magnetic particles; or
- the immunoassay does not comprise magnetic particles.

12. The method of claim 1, wherein the method is further used for predicting the onset of preeclampsia, monitoring the progression of preeclampsia , monitoring the regression of preeclampsia, assessing efficacy of treatment for preeclampsia, identifying a sub-population of patients who should be treated for preeclampsia or identifying a sub-population of patients who should be monitored for preeclampsia symptoms.

13. A composition comprising one or more solid surfaces comprising
A) binding agents for CSNK1D, CUEDC1 and ZRANB2 PEE autoantibodies; or
B) binding agents for CSNK1D, SULT4A1 and Junction Plakoglobin PEE autoantibodies.

14. A diagnostic assay kit comprising:
c. reagents for detecting a PEE autoantibody in a biological sample from a pregnant woman;
d. a composition comprising one or more solid surfaces that contain one or more binding agents for the PEE autoantibody; and
e. instructions for use of the assay.

15. The kit of claim 14, wherein the composition comprises
a solid surface capable of binding the CSNK1D, CUEDC1 and ZRANB2 PEE autoantibodies; or
a solid surface capable of binding the CSNK1D, SULT4A1 and Junction Plakoglobin PEE autoantibodies.
